(19) Europäisches Patentamt
European Patent Office
Office européen des brevets

(11) **EP 2 167 965 B1**

(12) **EUROPÄISCHE PATENTSCHRIFT**

(45) Veröffentlichungstag und Bekanntmachung des Hinweises auf die Patenterteilung:
**10.04.2013  Patentblatt 2013/15**

(21) Anmeldenummer: 08761286.7

(22) Anmeldetag: **20.06.2008**

(51) Int Cl.:
***G01N 33/543*** *(2006.01)*

(86) Internationale Anmeldenummer:
**PCT/EP2008/057913**

(87) Internationale Veröffentlichungsnummer:
**WO 2009/000784 (31.12.2008 Gazette 2009/01)**

(54) **VERFAHREN ZUR DETEKTION VON ANALYTEN**

METHOD FOR DETECTING ANALYTES

PROCÉDÉ DE DÉTECTION D'ANALYTES

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **22.06.2007  DE 102007029766**
**04.08.2007  DE 102007037068**

(43) Veröffentlichungstag der Anmeldung:
**31.03.2010  Patentblatt 2010/13**

(73) Patentinhaber: **B.R.A.H.M.S GmbH**
**16761 Hennigsdorf (DE)**

(72) Erfinder:
• **BERGMANN, Andreas**
**12351 Berlin (DE)**
• **STRUCK, Joachim**
**13465 Berlin (DE)**

(74) Vertreter: **Kilger, Ute**
**Boehmert & Boehmert**
**Pettenkoferstrasse 20-22**
**80336 München (DE)**

(56) Entgegenhaltungen:
**EP-A- 0 303 229      WO-A-92/16841**
**US-A1- 2006 211 055    US-B1- 6 348 318**

**Beschreibung**

Gegenstand der vorliegenden Erfindung ist ein Verfahren zur Detektion von Analyten aus biologischen Proben umfassend die folgenden Verfahrensschritte:

a) Bereitstellung eines an eine Festphase immobilisierten Reversiblen Bindungspartners 1, an den ein Analyt-Binder über einen an den Analyt-Binder gebundenen Reversiblen Bindungspartner 2 reversibel gebunden ist, wobei der Analyt-Binder immobilisiert wird durch Bindung zwischen den Reversiblen Bindungspartnem 1 und 2,

b) Hinzugabe der biologischen Probe und Bindung des Analyten an den reversibel immobilisierten Analyt-Binder im Fall, dass die biologische Probe den Analyten enthält,

c) Abtrennung der biologischen Probe,

d) Zugabe eines Ablösepuffers, der die Bindung zwischen den Reversiblen Bindungspartnern 1 und 2 löst, wobei die Bindung des Analyten an den Analyt-Binder optional bestehen bleibt, und wobei der Ablösepuffer eine weitere für die Detektion erforderliche markierte Komponente enthält, die ein zweiter Analyt-Binder für die Durchführung eines Sandwichimmunoassays ist, und

e) Detektion des Analyten im Ablösepuffer im Fall, dass die biologische Probe den Analyten enthält beziehungsweise Feststellen der Abwesenheit des Analyten im Fall, dass die biologische Probe den Analyten nicht enthält.

Schnelltest-, oder point-of-care (POC) Testverfahren sind in diversen Technologien entwickelt worden (1). Die wenigsten davon haben allerdings Marktreife erlangt.

Heutzutage etablierte Schnelltestverfahren, wie z.B. das TRIAGE System (Biosite, San Diego, USA), insbesondere aber das am weitesten verbreitete Verfahren, die Immunchromatographie, weisen nachteilig gegenüber klassischen - sei es manuell oder automatisiert abzuarbeitenden - Immunoassays eine geringere analytische Sensitivität sowie verminderte Präzision auf, können aber vorteilhaft, jedenfalls in geeigneten Ausführungsformen, unprozessierte Vollblutproben verarbeiten (2)-(7).

Die Nachteile gründen sich vor allem auf die Probenmatrixabhängigkeit der Verfahren und die Limitierung des verwendbaren Probevolumens.

Aufgabe der vorliegenden Erfindung ist es daher, ein u.a. als Schnelltest durchzuführendes Verfahren zu entwickeln, das unprozessierte Vollblutproben verarbeiten kann und analytische Sensitivität und Präzision aufweist, die mit denen von klassischen Immunoassays vergleichbar ist.

Gegenstand der vorliegenden Erfindung ist insbesondere die Verwendung eines erfindungsgemäßen reversiblen Bindungssystems für die Immobilisierung eines analytspezifischen Binders, z.B. anti-PCT-Antikörpers.

Gegenstand der vorliegenden Erfindung ist somit ein Verfahren zur Detektion von Analyten aus biologischen Proben umfassend die folgenden Verfahrensschritte:

a) Bereitstellung eines an eine Festphase immobilisierten Reversiblen Bindungspartners 1, an den ein Analyt-Binder über einen an den Analyt-Binder gebundenen Reversiblen Bindungspartner 2 reversibel gebunden ist, wobei der Analyt-Binder immobilisiert wird durch Bindung zwischen den Reversiblen Bindungspartnem 1 und 2,

b) Hinzugabe der biologischen Probe und Bindung des Analyten an den reversibel immobilisierten Analyt-Binder im Fall, dass die biologische Probe den Analyten enthält,

c) Abtrennung der biologischen Probe,

d) Zugabe eines Ablösepuffers, der die Bindung zwischen den Reversiblen Bindungspartnern 1 und 2 löst, wobei die Bindung des Analyten an den Analyt-Binder optional bestehen bleibt, und wobei der Ablösepuffer eine weitere für die Detektion erforderliche markierte Komponente enthält, die ein zweiter Analyt-Binder für die Durchführung eines Sandwichimmunoassays ist, und

e) Detektion des Analyten im Ablösepuffer im Fall, dass die biologische Probe den Analyten enthält beziehungsweise Feststellen der Abwesenheit des Analyten im Fall, dass die biologische Probe den Analyten nicht enthält.

Dem Fachmann ist klar, dass zwischen dem Schritt b) und c) eine gewisse Inkubationszeit einzuhalten ist. Die Inkubationszeit sollte nicht weniger als 30 s und nicht länger als 24 h betragen, besonders bevorzugt im Rahmen eines Schnelltestverfahrens ist eine Inkubationszeit zwischen 5 und 15 min. Der Ablöseprozess gemäß Schritt d) kann bevorzugterweise bis 24 h betragen, bevorzugt im Rahmen eines Schnelltestverfahrens ist der Ablöseprozess länger als 15 s und kürzer als 15 min, bevorzugt zwischen 5 bis 15 min.

Die Bindung des Analyten an den Analyt-Binder bleibt während und/oder nach der Ablösung gemäß d) bestehen oder nicht. Beide Optionen sind erfindungsgemäß denkbar. Bevorzugt bleibt bei und/oder nach Zugabe des Ablösepuffers nach d) die Bindung zwischen dem Analyten und dem Analyt-Binder bestehen. Die Bindung bleibt bevorzugt zu mindestens 90% der Analytenmoleküle bestehen. Falls die Bindung nicht bestehen bleibt, wird sie bevorzugt zu einem späteren Zeitpunkt wieder hergestellt. Am meisten bevorzugt besteht zum Zeitpunkt der Detektion die Bindung zwischen Analyt und Analyt-Binder, das heißt, dass sie entweder während des Ablöseprozesses erhalten bleibt beziehungsweise wieder hergestellt wird.

In einer bevorzugten Ausführungsform ist der Analyt-Binder ein anti-Analyt Antikörper.

In einer bevorzugten Ausführungsform des erfindungsgemäßen Verfahrens ist die biologische Flüssigkeit eine unprozessierte Vollblutprobe.

Erfindungsgemäß kann der immobilisierte Reversible Bindungspartner 1 direkt oder mittels eines Trägerproteins immobilisiert werden. Ein beispielhaftes Trägerprotein ist BSA (bovines Serumalbumin). Dem Fachmann sind geeignete Trägerproteine bekannt.

Erfindungsgemäß sind verschiedene Bindungspartner geeignet, die feste Bindungen bilden können, unter bestimmten Bedingungen jedoch diese Bindung aufgeben können und somit ein "reversibles Bindungssystem" darstellen. Von besonderem Interesse sind hier Bindungssysteme, bei denen die Bindung durch relativ milde Bedingungen destabilisiert werden kann, also Bedingungen, welche die Konformation von Proteinen, im Besonderen Antikörpern, und deren Bindung mit einem Antigen nicht wesentlich beeinträchtigen.

Die Anwendung solcher reversibler Bindungssysteme ist bekannt im Zusammenhang von Reinigungsmöglichkeiten rekombinanter Proteine aus komplexen Proteingemischen wie etwa Zellextrakten. Mittels rekombinanter DNA Technologie wird in diesem Zusammenhang der für das interessierende Protein kodierenden cDNA Sequenz eine Sequenz angefügt, die für ein sogenanntes "tag" kodiert, so dass ein um ein "tag" verlängertes Protein exprimiert wird. Über einen an eine Festphase immobilisierten Bindungspartner für das "tag" kann dann das "tag"-Protein aus dem Proteingemisch selektiv gebunden und anschließend durch eine spezifische Destabilisierung der "tag"-Bindung wieder von der Festphase abgetrennt werden. Das Protein von Interesse liegt dann angereichert oder rein in Lösung vor. Übersichten zu gebräuchlichen "tags", korrespondierenden immobilisierten Bindungspartnern und spezifischen Destabilisierungsmethoden finden sich z.B. bei **(8)-(10)**.

Solche Bindungssysteme können im Rahmen der gegenwärtigen Erfindung als Bindungspartner verwendet werden.

Im Rahmen des erfindungsgemäßen Verfahrens kann insbesondere das Bindungspaar Reversibler Bindungspartner 1 und 2 ausgewählt sein aus einem der folgenden Bindungspaare:

a) positiv und negativ geladene Peptidoligomere,

b) $Ca^{2+}$-bindendes Peptid/Protein und Antikörper, der das Peptid/Protein mit größerer Affinität bindet, wenn das Peptid/Protein $Ca^{2+}$ gebunden hat,

c) Oligohistidin (z.B. 6His) und Ni-NTA,

d) Biotin und Avidin oder Streptavidin oder Neutravidin.

Einige reversible Bindungssysteme, die im Rahmen der vorliegenden Erfindung liegen, seien hier kurz vorgestellt:

Ein Beispiel für ein $Ca^{2+}$-bindendes Peptid/Protein und einen Antikörper, der das Peptid/Protein mit größerer Affinität bindet, wenn das Peptid/Protein $Ca^{2+}$ gebunden hat ist das FLAG/M1 System, wobei das $Ca^{2+}$-bindende Peptid/Protein ein FLAG-Peptid und der Antikörper ein M1 Antikörper ist. Bei einem anderen System ist das $Ca^{2+}$-bindende Peptid/Protein ein Protein C Peptid und der Antikörper ein HPC4 Antikörper ist.

**a) FLAG/M1**

Ein Beispiel ist somit die Bindung zwischen dem monoklonalen Antikörper M1 (auch als 4E11 beschrieben) und dem sogenannten FLAG-Peptid. Dieses Peptid kann Calcium$^{2+}$ binden und nimmt dadurch eine bestimmte Konformation an. Nur diese Konformation wird effizient von M1 gebunden. Durch Zugabe von Calcium$^{2+}$-komplexierender Agentien wie etwa EDTA wird dem Peptid Calcium$^{2+}$ entzogen, das Peptid nimmt eine andere Konformation ein, wodurch der M1 Antikörper seine Affinität zum Peptid drastisch vermindert; US Pat 4,851,341 **(11)**.

### b) Protein C/HPC4

Ein weiteres, analoges Beispiel eines solchen Bindungspaares ist ein von humanem Protein C abgeleitetes Peptid, das ebenfalls eine Calcium$^{2+}$-abhängige Konformation einnehmen kann, und der monoklonale Antikörper HPC4 **(12)**.

### c) Ni2+-NTA/ 6 His

Ein weiteres Beispiel ist das Ni$^{2+}$-NTA/ 6 His-System, das spezifisch durch Imidazol Zugabe destabilisiert werden kann **(13)**.

### d) Geladene Peptidoligomere

Ein weiteres Beispiel ist ein Bindungspaar von positiv bzw. negativ geladenen Peptidoligomeren, wie z.B. Oligo-Lys bzw. Oligo-Asp, dessen Bindung miteinander durch Erhöhung der Ionenstärke destabilisiert werden kann. Als Bindungspaar beschrieben ist ein geladenes Peptidoligomer in Verbindung mit einer Ionenaustauschermatrix; auch diese Bindung ist durch Erhöhung der Ionenstärke zu destabilisieren **(14)-(16)**.

Neben reversiblen Bindungssystemen, bei denen einer der Bindungspartner in ein rekombinantes Protein integriert ist, sind auch Systeme bekannt, bei denen einer der Bindungspartner chemisch an das zu immobilisierende Protein konjugiert wird. Zu nennen ist hier beispielsweise das Biotin/Avidin-System. Biotin kann z.B. in Form eines NHS-Esters an primäre Ammgruppen eines Proteins konjugiert werden **(17)**. Dieses Bindungssystem ist durch Zugabe eines Überschusses an Biotin auch zu destabilisieren, jedoch nur unter drastischen Bedingungen wie erhöhter Temperatur und längerer Inkubationszeit **(18)**. Es ist jedoch eine Variante dieses Systems beschrieben, welche sogenanntes monomeres Avidin verwendet; nach Absättigung "nichtreversibler" Bindungsstellen mit Biotin, sind die verbleibenden "reversiblen" Bindungsstellen geeignet, ein biotinyliertes Protein zu binden, das dann unter milden Bedingungen mittels Biotin aus der Bindungsstelle gelöst werden kann **(19)**.

Peptid-"tags", wie oben für rekombinante Proteine beschrieben, sollten auch chemisch an ein Protein konjugierbar sein, womit das so derivatisierte Protein dann einer reversiblen Bindung zugeführt werden kann. Im Rahmen der vorliegenden Erfindung ist die chemische Konjugation als bevorzugte Variante der Protein-Derivatisierung anzusehen, da der Derivatisierungsgrad steuerbar ist und die chemische Konjugation vergleichsweise zeitsparend und kostengünstig ist.

Alle bisher genannten Beispiele reversibler Bindungssysteme basieren auf einer nicht-kovalenten Wechselwirkung der Bindungspartner. Im Rahmen der vorliegenden Erfindung sind als reversible Bindungssysteme jedoch auch solche Systeme anzusehen, bei denen zunächst eine kovalente Bindung vorliegt, die dann chemisch gelöst werden kann. Beispielhaft zu nennen ist hier die kovalente Vernetzung mittels eines spaltbaren hetero-bifunktionalen Crosslinkers wie etwa SPDP (20); durch Zugabe eines reduzierenden Agens kann die Disulfidbrücke gespalten und die Bindung damit gelöst werden.

Gegenstand der Erfindung ist somit auch das erfindungsgemäße Schnelltestverfahren, wobei der Analyt-Binder kovalent an den Reversiblen Bindungspartner 2 gebunden ist.

Gegenstand der vorliegenden Erfindung ist somit in einer bevorzugten Ausführungsform das erfindungsgemäße Verfahren, wobei der Analyt-Binder ein Anti-Procalcitonin Antikörper ist.

Ein bevorzugter Gegenstand der Erfindung ist somit die Anwendung eines reversiblen Bindungssystems für die Immobilisierung eines Analyt-spezifischen Binders, z.B. anti-PCT-Antikörpers.

Die Anwendung wird im Folgenden beispielhaft für das reversible Bindungssystem der geladenen Peptidoligomere (s. o.) formuliert, ist für die anderen Systeme aber analog zu übertragen. Oligo-Asp wird an ein Trägerprotein wie Bovines Serumalbumin (BSA) konjugiert, und dieses Konjugat wird stabil auf eine Festphase wie z.B. hochbindende Polystyrol-Mikrotiterplatten immobilisiert. Oligo-Lys wird an einen anti-PCT Antikörper konjugiert. Inkubation des Oligo-Lys-anti-PCT Antikörpers mit der Festphase führt zu einer Immobilisierung des Antikörpers durch Bindung zwischen den Oligo-Lys- und Oligo-Asp-Anteilen durch ionische Wechselwirkung.

In einer alternativen Ausführungsform kann der anti-PCT Antikörper auch indirekt mittels eines Oligo-Lys-derivatisierten Antikörpers gegen den anti-PCT Antikörper immobilisiert werden (beispielweise ein anti-Maus IgG Antikörper, wenn es sich bei dem anti-PCT Antikörper um einen Maus-monoklonalen Antikörper handelt).

Das Prinzip der Immunextraktion wird im Folgenden für die PCT Analyse beispielhaft erläutert:

Zunächst wird ein anti-PCT Antikörper über ein reversibles Bindungssystem auf eine Festphase immobilisiert. Eine zu untersuchende Vollblutprobe (für das Beispiel geladene Peptidoligomere und EDTA-Blut) wird hinzugegeben, und PCT wird aus der Probe immunextrahiert. Dabei kann ein vergleichsweise großes Volumen an Probe verwendet werden, so dass eine vergleichsweise große Menge an PCT extrahiert werden kann, was im Folgenden zu einer hohen analytischen Sensitivität des Tests führt. Nach einer kurzen Inkubationszeit wird die Probe abgetrennt (durch Waschen oder andere geeignete Trennmethoden wie etwa Absaugen oder geeignetes Zentrifugieren). Im nächsten Schritt wird ein Puffer zugesetzt ("Ablösungspuffer"), der ein Agens enthält (im Oligo-Lys-/Oligo-Asp-System z.B. das stark negativ geladene Heparin), das geeignet ist, die Bindung des Antikörpers an die Festphase, nicht jedoch die Bindung zwischen PCT und Antikörper weitgehend zu destabilisieren. Somit steht eine Lösung zur Verfügung, die den zu bestimmenden Analyten (komplexiert an einen spezifischen Antikörper) in großer Menge enthält.

Die Lösung ist für jede untersuchte Probe immer von identischer Beschaffenheit, da die Probenmatrix vorher abgetrennt wurde. Die Vorteile dieses Schritts bestehen also in:

- der Möglichkeit, unprozessiertes Vollblut zu verwenden,

- der Möglichkeit, größere Probevolumina zu verwendenund damit eine relativ große Menge des Analyten zu extrahieren und schließlich eine hohe analytische Assaysensitivität im sich anschließenden Bestimmungsverfahren zu erreichen,

- der Matrixunabhängigkeit für Folgeschritte im Bestimmungsverfahren, die sich vorteilhaft auf Präzision und Richtigkeit des Bestimmungsverfahrens auswirken.

Der immunextrahierte und in Lösung gebrachte Analyt kann durch verschiedene Methoden detektiert werden. Ein Beispiel ist die TRACE Technologie, eine andere (weiter unten erläuterte Methode) die Immunchromatographie. Die TRACE Technologie verwendet für einen Sandwichimmunoassay zwei Antikörper, die mit unterschiedlichen Fluoreszenzlabeln markiert sind (beispielsweise Cyanin bzw. Kryptat). Bei Sandwichbildung mit dem Analyten in Lösung erreichen beide Label eine räumliche Nähe, die zu einer spezifischen Lichtemission nach entsprechender Licht-Anregung führt. Im beispielhaften Zusammenhang bei der Analyse von PCT wird der mit Oligo-Lys konjugierte anti-PCT Antikörper vor der Immobilisierung zunächst außerdem mit Kryptat markiert. Hiermit wird die Immunextraktion durchgeführt. Im Ablösungspuffer ist nun neben dem Ablöse-Agens der zweite für die Sandwichbildung nötige, Cyanin-markierte anti-PCT Antikörper enthalten. Damit kommt es durch Zugabe des Ablösungspuffers sowohl zur Ablösung des Antigen-Antikörper-Komplexes von der Festphase als auch zur Sandwichbildung, die nach geeigneter Lichtanregung detektierbar ist. Ablösung und Zugabe des zweiten Antikörpers zur Sandwichbildung können auch sequentiell erfolgen.

In einer oben beschrieben Variante kann der Analyt-spezifische Antikörper vor der Immunextraktion indirekt über einen Oligo-Lys-derivatisierten z.B. anti-Maus IgG Antikörper immobilisiert sein. In dieser Ausführungsform kann das Fluoreszenzlabel dann an den einen oder den anderen Antikörper konjugiert sein.

Beim Beispiel der Immunchromatographie wird der mit Oligo-Lys konjugierte anti-PCT Antikörper vor der Immobilisierung zunächst außerdem mit Biotin markiert. Hiermit wird die Immunextraktion durchgeführt. Im Ablösungspuffer ist nun neben dem Ablöse-Agens der zweite für die Sandwichbildung nötige, mit kolloidalem Gold markierte anti-PCT Antikörper enthalten. Ablösung und Zugabe des zweiten Antikörpers zur Sandwichbildung können auch sequentiell erfolgen. Das Reaktionsgemisch wird anschließend auf einen immunchromatographischen Teststreifen appliziert, auf den als Fänger für den gebildeten Sandwich ein Biotin-Binder wie z.B. Avidin als feine Linie im hinteren Bereich des Teststreifens quer zur Laufrichtung der Reaktionslösung aufgesprüht ist.

In einer besonders bevorzugten Ausführungsform des erfindungsgemäßen Verfahrens ist der Analyt-Binder markiert und bleibt auch nach Zugabe des Ablösepuffers markiert mit einem Label für die Detektion des Analyten.

Bei der Ausführung Immunochromatographie können folgende weitere Varianten denkbar sein:

Der Immunextraktionsantikörper ist neben der Derivatisierung mit Bindungspartner 2 zusätzlich biotinyliert. Ein zweiter Antikörper für die Sandwichbildung ist markiert (z.B. mit kolloidalem Gold). Der Fänger auf dem Teststreifen wäre dann ein Biotin-Binder wie Avidin oder Streptavidin oder Neutravidin.

Der Immunextraktionsantikörper (z.B. Schaf polyklonal) ist nur mit Bindungspartner 2 derivatisiert. Ein zweiter Antikörper für Sandwichbildung müsste aus einer anderen Tierspezies stammen (z.B. Maus monoklonal) und ist markiert (z.B. mit kolloidalem Gold).

Der Fänger auf dem Teststreifen wäre dann ein anti-Schaf IgG Antikörper. Beide Varianten sind auch in der Form vorstellbar, dass der Immunextraktionsanrikörper die Markierung trägt und wobei in der ersten Variante dann der zweite Antikörper biotinyliert ist (alternativ könnte hier der zweite Antikörper auch direkt auf dem Streifen aufgesprüht sein).

In der am meisten bevorzugten Ausführungsform des Verfahrens enthält der Ablösepuffer eine weitere für die Detektion erforderliche markierte Komponente, die ein zweiter Analyt-Binder für die Durchführung eines Sandwichimmunoassays ist.

Besonders bevorzugt erfolgt die Detektion des markierten Analyten durch einen Immunoassay unter Verwendung der TRACE Technologie.

In einer anderen bevorzugten Ausführungsform erfolgt die Detektion des markierten Analyten mittels Immunochromatographie.

In einer erfindungsgemäß besonders bevorzugten Variante wird die biologische Probe unverdünnt und in einem solchen Volumen eingesetzt wird, dass der beschichtete Anteil der Festphase vollständig in Kontakt mit der biologischen Probe gebracht wird.

Besonders bevorzugt ist, dass die Dauer von Zugabe der Probe bis zur Detektion 30 min nicht überschreitet und das Verfahren somit als Schnelltestverfahren anzusehen ist.

Gleichfalls Gegenstand der vorliegenden Erfindung ist ein Kit zur Durchführung des erfindungsgemäßen Verfahrens umfassend:

- eine Festphase mit einem immobilisierten Reversiblen Bindungspartner 1,

- Komplex umfassend Analyt-Binder gebunden an den Reversiblen Bindungspartner 2, wobei dieser Komplex optional bereits durch Bindung der Reversiblen Bindungspartner 1 und 2 auf der Festphase immobilisiert vorliegen kann,

- Ablösepuffer, der die Bindung zwischen den Reversiblen Bindungspartnern 1 und 2 löst, wobei jedoch die Bindung des Analyten an den Analyt-Binder optional bestehen bleibt, und wobei der Ablösepuffer eine weitere für die Detektion erforderliche markierte Komponente enthält, die ein zweiter Analyt-Binder für die Durchführung eines Sandwichimmunoassays ist.

In einer besonders bevorzugten Ausführungsform ist der im Kit befindliche Analyt-Binder ein Anti-PCT Antikörper.

Der am meisten bevorzugte Kit enthält als ausgewähltes Bindungspaar die Reversiblen Bindungspartner 1 und 2 ausgewählt aus einem der folgenden Bindungspaare:

- positiv und negativ geladene Peptidoligomere,

- $Ca^{2+}$-bindendes Peptid/Protein und Antikörper, der das Peptid/Protein mit größerer Affinität bindet, wenn das Peptid/Protein $Ca^{2+}$ gebunden hat,

- Oligohistidin (z.B. 6His) und Ni-NTA,

- Biotin und Avidin oder Streptavidin oder Neutravidin.

Die folgenden Beispiele sollen die Erfindung näher erläutern ohne den Gegenstand der Erfindung einzuschränken:

**Beispiele**

**Immunextraktion von PCT aus Vollblut**

Als Grundlage für die Bearbeitung weiterer Schritte wurde zunächst geprüft, ob und wie effektiv sich ein Analyt, hier am Beispiel PCT untersucht, aus unprozessiertem EDTA-Vollblut mittels eines an eine Festphase immobilisierten Analyt-spezifischen Antikörpers extrahieren lässt. Nach kurzer Inkubation (5 min) und Abtrennung der Vollblutprobe wurde das gebundene PCT mit einem zweiten, chemilumineszenzmarkierten anti-PCT Antikörper nachgewiesen. Zum Vergleich wurde PCT-enthaltendes Serum, verdünnt in Puffer, auf der gleichen Festphase inkubiert, jedoch länger (2 h), und das gebundene PCT wurde nach Abtrennung der verdünnten

Probe wie oben nachgewiesen.

Im Einzelnen wurde der Versuch wie folgt durchgeführt.:

Zur Bestimmung der Immunextraktion wurden die Komponenten aus dem BRAHMS PCT sensitiv LIA Kit verwendet (BRAHMS Aktiengesellschaft, Hennigsdorf, Deutschland). Der lumineszenzmarkierte anti-PCT Tracerantikörper A wurde in Tracerrekonstitutionspuffer B nach Arbeitsanleitung gelöst. Eine Standardreihe wurde in EDTA-Vollblut eines gesunden Blutspenders zu folgenden Konzentrationen gelöst: c = 0,007; 0,014; 0,036; 0,171; 0,383; 1,73; 7,99 ng/ml. In die mit anti-PCT Antikörper beschichteten Röhrchen wurden je 300 μl Vollblutstandards pipettiert und 5 min bei Raumtemperatur inkubiert. Danach wurden die Röhrchen mit 5x1 ml Waschlösung (8 mM Tris, 60 mM NaCl, 0,2% Tween 20, pH 7,5) gewaschen. Anschließend wurden 200 μl lumineszenzmarkierter anti-PCT Antikörper pipettiert und 2 h bei Raumtemperatur inkubiert; die Röhrchen wurden nochmals mit 5x1 ml Waschlösung gewaschen. Die an den Röhrchen gebundene Chemilumineszenz wurde in einem Luminometer (Firma BERTHOLD, BAD WILDBAD, DEUTSCHLAND, LB952T; Basisreagenzien BRAHMS AG) vermessen.

Die Referenzmethode (Bestimmung von PCT aus verdünntem Serum) wurde auf Basis des BRAHMS PCT sensitiv LIA Kits (BRAHMS Aktiengesellschaft, Hennigsdorf, Deutschland) wie folgt durchgeführt: Eine Standardreihe wurde in Nullserum aus dem Testkit zu folgenden Konzentrationen gelöst:

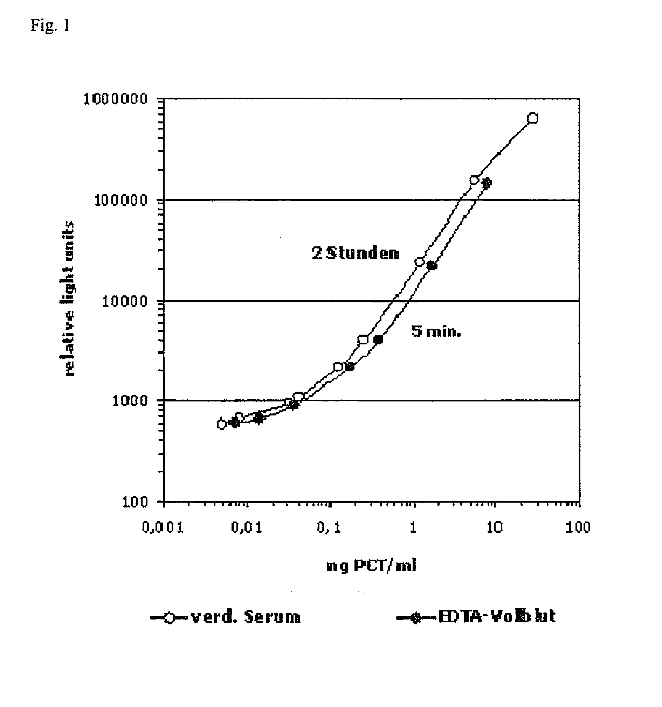

c - 0; 0,008; 0,031; 0,042; 0,126; 0,251; 1,21; 5,61; 28,0 ng/ml. In die mit anti-PCT Antikörper beschichteten Röhrchen wurden je 50 $\mu$l Serumstandards und 250 $\mu$l Tracerrekonstitutionspuffer B pipettiert und 2 h bei Raumtemperatur inkubiert. Danach wurden die Röhrchen mit 5x1 ml Waschlösung (8 mM Tris, 60 mM NaCl, 0,2% Tween 20, pH 7,5) gewaschen. Anschließend wurden 200 $\mu$l lumineszenzmarkierter anti-PCT Antikörper pipettiert und 2 h bei Raumtemperatur inkubiert; die Röhrchen wurden nochmals mit 5x1 ml Waschlösung gewaschen. Die an den coated tubes gebundene Chemilumineszenz wurde in einem Luminometer (Firma BERTHOLD, BAD WILDBAD, DEUTSCHLAND, LB952T; Basisreagenzien BRAHMS AG) vermessen (Fig. 1).

**Darstellung von reversibel an eine Festphase zu bindenden Antikörpern**

Mehrere der weiter oben beschriebenen reversiblen Bindungssysteme wurden auf ihre Eignung untersucht, einen Antikörper, hier am Beispiel eines anti-PCT Antikörpers, reversibel an eine Festphase zu binden. Dazu wurde der Antikörper unterschiedlich derivatisiert (s. Tabelle 1) und mit einem Chemilumineszenz-Label markiert. Damit wurde untersucht, wie sich diese Antikörper an zum jeweiligen Derivat korrespondierende Festphasen binden und wieder ablösen lassen. Die Zusammenfassung der Ergebnisse in Tabelle 1 zeigt, dass sich alle untersuchten reversiblen Bindungssysteme mit Ausnahme des Biotin/Avidin-Systems spezifisch und schnell destabilisieren lassen und - mit Einschränkungen für das 6His/Ni-NTA-System - unbeeinträchtigt sind durch Blutmatrix.

Tabelle 1

| Derivat am Antikörper (Bindungspartner 1) | Festphase (Bindungspartner 2) | Ablösungsagens | Anteil des unspezifisch durch Blut (10 min Inkubation) ablösbaren Antikörpers bezogen auf die vorher gebundene Menge | Anteil des durch Ablösungsagens (10 min Inkubation) ablösbaren Antikörpers bezogen auf die vorher gebundene Menge | Anteil des durch Ablösungsagens (30 min Inkubation) ablösbaren Antikörpers bezogen auf die vorher gebundene Menge |
|---|---|---|---|---|---|
| Biotin | Avidin | 25 mM Biotin | 0,7% | 1,9% | 2,5% |
| M1 Antikörper | FLAG-Peptid-BSA | 10 mM ED-TA | 7,8% | 58,7% | 74,8% |
| 6His | Ni-NTA | 250 mM Imidazol | 38,6% | 89,4% | 98,8% |
| HPC4 Antikörper | Protein C-Peptid-BSA | 10 mM E-DTA | 3,5% | 38,3% | 69,2% |
| Oligo-Asp | Oligo-Lys BSA | 0,01% Heparin | 5,3% | 80,0% | 93,2% |

Es wurde weiterhin beobachtet, dass die Effizienz von Bindung und spezifischer Ablösung beeinflusst werden kann durch den jeweiligen Derivatisierungsgrad (das molare Verhältnis von

Bindungspartner 1 pro Antikörper), der Konzentration des auf der Festphase immobilisierten Bindungspartners 2, dadurch, ob Bindungspartner 1 an den Antikörper konjugiert und Bindungspartner 2 immobilisiert war, oder Bindungspartner 2 an den Antikörper konjugiert und Bindungspartner 1 immobilisiert war, und im Fall des Oligo-Asp/Oligo-Lys-Systems die Länge beider Oligomere. Daher fallen im Rahmen dieser Erfindung derartige Varianten zwar auch unter den Begriff der reversiblen Bindungssysteme und die Ansprüche schließen derartige Varianten ausdrücklich mit ein, jedoch sind die hier beispielhaft beschriebenen Varianten als bevorzugte Ausführungsformen anzusehen. Als Alternative zu Oligo-Lys-BSA erwiesen sich auch Polylysin-Festphasen als geeignet.

Im Einzelnen wurde der Versuch wie folgt durchgeführt:

**Bindungspartner 1 (Flüssigphase)**

**1. Herstellung von MACN-chemlumineszenz-markiertem Antikörper**

Ein polyklonaler anti-Calcitonin Schaf Antikörper wurde wie folgt behandelt:

Es wurden drei Markierungen angesetzt; diese wurden nach der Inkubation unterschiedlich umgepuffert (s.u.).

Zur Chemilumineszenzmarkierung des Antikörpers wurden 1 ml der Antikörperlösung (c=3,19 mg/ml) mit 40 $\mu$l 1M Kaliumphosphat, pH 7,8, und mit 2,7 $\mu$l MACN-Akridinium-NHS-Ester (c=1 mg/ml; Firma InVent GmbH, Hennigsdorf, Deutschland) versetzt (molares Markierungsverhältnis Antikörper: MACN 10:1). Dann wurde 30 min bei Raumtemperatur inkubiert. Anschließend wurden die Markierungsansätze über NAP-10 Gelfiltrationssäulen (Pharmacia, Upsalla, Schweden) in 1,5 ml Laufmittel umgepuffert und dabei von niedermolekularen Bestandteilen befreit:

Markierung 1 PBS pH 7,4 für Biotinylierung siehe 2.1.
Markierung 2 100 mM Natriumphosphat, 5 inM EDTA, pH 6,9 für SPDPAktivierung siehe 2.2.
Markierung 3 PBS, 10 mM EDTA, pH 8,0 für SMCC-Aktivierung siehe 2.3.

Der Proteingehalt wurde photometrisch bestimmt, die markierten Antikörper wurden portioniert bei -20°C gelagert.

**2. Derivatisierungen der MACN-chemilumineszenz-markierten Antikörper**

**2.1. Biotinylierung**

Der MACN-Antikörper wurde mit EZ-Link NHS-Chromogenic-Biotin (Firma Pierce, Rockford, IL, USA, Art. Nr. 21325) im molaren Verhältnis von 1:10 biotinyliert.

1 mg MACN-Antikörper wurde mit 5,41 $\mu$l 1,233 mM Biotin-Lösung (frisch gelöst in DMSO) versetzt und 60 min bei Raumtemperatur inkubiert. Die Reaktion wurde mit 100 $\mu$l 50 mM Glycin 10 min bei Raumtemperatur gestoppt und über eine NAP5-Säule (Pharmacia, Upsalla, Schweden) aufgereinigt.

Zur Abtrennung letzter Reste von nicht an Antikörper gebundenem Biotin wurde eine Gelfiltrations-HPLC durchgeführt (Säule: Bio-Sil Sec 400, Firma Biorad, München, Deutschland). Die Probe wurde aufgetragen und bei einer Flussrate von 0,8 ml/min mit PBS, pH 7,4 chromatographiert. Mit einem Durchflussphotometer wurden die Wellenlängen 280 nm und 354 nm gemessen. Der Markierungsgrad $\mu$M Biotin/$\mu$M Antikörper betrug am Peak 0,48. Die Antikörper enthaltenden Fraktionen (Retentionszeit 11-12 min) wurden gepoolt.

Der Proteingehalt wurde photometrisch bestimmt, das Konjugat wurde portioniert bei -20°C gelagert.

**2.2. Konjugation mit anti FLAG-tag MI-Antikörper, bzw. anti ProtC-tag HPC4 Antikörper**

Für die Konjugation mit anti FLAG-tag M1 (Firma Sigma, Deisenhofen, Deutschland, Art. Nr. F 3040) -, bzw. anti ProtC-tag HPC4 (Firma Roche, Nutley, NJ, USA, USA, Art. Nr. 11814516001) Antikörper wurden diese und der MACN Antikörper mit SPDP (N-succinimidyl 3-(2-pyridyldithio) propionate, Firma Pierce, Rockford, IL, USA, Art. Nr. 21857) im molaren Verhältnis von 1:7 aktiviert (siehe "Instructions SPDP Reagents", Pierce, Rockford, IL, USA).

Je 2 mg Antikörper wurden mit je 4,67 $\mu$l 20 mM SPDP (frisch gelöst in Ethanol) versetzt und 30 min bei Raumtemperatur inkubiert. Nur die aktivierten M1 und HPC4 Antikörper wurden mit 200 mM DTT (Dithiothreitol, gelöst in 100 mM Natriumphosphat, 5 mM EDTA, pH 6,9) auf eine Endkonzentration von 10 mM DTT eingestellt und 15 min bei Raumtemperatur reduziert.

Die reduzierten M1, bzw. HPC4 Antikörper wurden jeweils über eine NAP10-Säule gereinigt (Laufmittel: 100 mM Natriumphosphat, 5 mM EDTA, pH 6,9) und der Proteingehalt photometrisch bestimmt.

Die reduzierten Produkte wurden jeweils mit dem nicht reduzierten SPDP-aktivierten MACN-Antikörper im molaren Verhältnis von 1:1 über Nacht bei 4°C konjugiert. Anschließend wurde die Reaktion je mit 50 μl 100 mM Cystein 10 min gestoppt.

Der Proteingehalt wurde photometrisch bestimmt, die Konjugate wurden portioniert bei -20°C gelagert.

### 2.3. Konjugation mit His-tag, bzw. Oligo-Asp Peptid

Zur Kopplung des His-tag Peptids "PRG12" (Aminosäuresequenz RGSHHHHHHGGC (SEQ ID NO. 1), Firma JPT GmbH, Berlin, Deutschland, M=1359,8), bzw. des Oligo-Asp Peptids "D14C" (Aminosäuresequenz DDDDDDDDDDDDDDC (SEQ ID NO. 2), Firma JPT GmbH, Berlin, Deutschland, M=1731,43) wurde der MACN-Antikörper mit SMCC (Succinimidyl 4- (N-maleimidomethyl) cyclohexane-1-carboxylate, Firma Pierce, Rockford, IL, USA, Art. Nr. 22360) im molaren Verhältnis von 1:50 aktiviert (siehe "Instructions SMCC and SulfoSMCC", Pierce, Rockford, IL, USA).

2 mg MACN-Antikörper wurden mit 6,68 μl 100 mM SMCC (frisch gelöst in DMSO) versetzt und 30 min bei Raumtemperatur inkubiert. Das Produkt wurde über eine NAP10-Säule (Pharmacia, Upsalla, Schweden) gereinigt (Laufmittel: PBS, 10 mM EDTA, pH 8) und der Proteingehalt photometrisch bestimmt.

Anschließend erfolgte sofort die Konjugation mit dem His-tag, bzw. Oligo-Asp Peptid (gelöst in dest. Wasser) im molaren Verhältnis von 1:100. Nach Inkubation von 60 min bei Raumtemperatur wurde die Reaktion mit 100 μl 100 mM Cystein 10 min gestoppt, nochmals je über eine NAP25-Säule (Pharmacia, Upsalla, Schweden) gereinigt (Laufmittel: PBS, pH 7,4) und der Proteingehalt photometrisch bestimmt.

Beide Konjugate wurden aliquotiert bei -20°C gelagert.

### Bindungspartner 2 (Festphase)

### 1.1 Konjugation von BSA mit FLAG-tag / ProtC-tag

Zur Kopplung des Flag-tag Peptids "PDC12" (Aminosäuresequenz DYKDDDDKGGGC, (SEQ ID NO. 3), Firma JPT GmbH, Berlin, Deutschland, M=1286,46), bzw. ProtC-tag Peptids PEG15 (Aminosäuresequenz EDQVDPRLIDGKGGC, (SEQ ID NO. 4), Firma JPT GmbH, Berlin, Deutschland, M= 1601,7) wurde proteasefreies Bovines Serum Albumin (Firma Sigma, Deisenhofen, Deutschland) mit SMCC (Succinimidyl 4-(N-maleimidomethyl) cyclohexane-1-carboxylate, Firma Pierce, Rockford, IL, USA, Art, Nr. 22360) im molaren Verhältnis von 1:2 aktiviert (siehe "Instructions SMCC and Sulfo-SMCC", Pierce, Rockford, IL, USA).

2500 μl 0,5% BSA (gelöst in PBS, 10 mM EDTA, pH 8) wurden mit 3,78 μl 100 mM SMCC (frisch gelöst in DMSO) versetzt und 30 min bei Raumtemperatur inkubiert. Das Produkt wurde über eine NAP25-Säule (Pharmacia, Upsalla, Schweden) gereinigt (Laufmittel: PBS, 10 mM EDTA, pH 8) und der Proteingehalt photometrisch bestimmt.

Anschließend erfolgte sofort die Konjugation mit dem FLAG-tag, bzw. ProtC-tag Peptid (gelöst in dest. Wasser) im molaren Verhältnis von 1:100. Nach Inkubation von 60 min bei Raumtemperatur wurden die Konjugate je mit 100 μl 100 mM Cystein 10 min gestoppt und nochmals je über eine NAP25-Säule (Pharmacia, Upsalla, Schweden) gereinigt (Laufinittel: PBS, pH 7,4); der Proteingehalt wurde photometrisch bestimmt.

Das BSA+FLAG-tagund BSA+ProtC-tag-Konjugat wurde aliquotiert bei -20°C gelagert.

### 1.2 Konjugation von BSA mit Oligo-Lys Peptid

Zur Kopplung des Oligo-Lys Peptids "K14C" (Aminosäuresequenz KKKKKKKKKKKKKKC, (SEQ ID NO. 5) Firma JPT GmbH, Berlin, Deutschland, M=1914,27) wurde proteasefreies Bovines Serum Albumin (Firma Sigma, Deisenhofen, Deutschland) mit SMCC im molaren Verhältnis von 1:25 aktiviert.

1000 μl 0,5% BSA (gelöst in PBS, 10 mM EDTA, pH 8) wurden mit 18,9 μl 100 mM SMCC (frisch gelöst in DMSO) versetzt und 30 min bei Raumtemperatur inkubiert. Das Produkt wurde über eine NAP10-Säule (Pharmacia, Upsalla, Schweden) gereinigt (Laufmittel: PBS, 10 mM EDTA, pH 8) und der Proteingehalt photometrisch bestimmt.

Anschließend erfolgte sofort die Konjugation mit dem Oligo-Lys Peptid (gelöst in dest. Wasser) im molaren Verhältnis von 1:100. Nach Inkubation von 60 min bei Raumtemperatur wurde die Reaktion mit 100 μl 100 mM Cystein 10 min gestoppt, nochmals über eine NAP25-Säule (Pharmacia, Upsalla, Schweden) gereinigt (Laufmittel: PBS, pH 7,4) und der Proteingehalt photometrisch bestimmt.

Das BSA+Oligo-Lys Konjugat wurde aliquotiert bei -20°C gelagert.

### 2. Kopplung der Konjugate, bzw. von Avidin

Bestrahlte Sternröhrchen (Firma Greiner, Frickenhausen, Deutschland) wurden mit den Konjugaten aus 1.1.

und 1.2., bzw. mit Avidin (Pierce, Rockford, IL, USA, Art. Nr. 21121, gelöst in dest. Wasser) wie folgt beschichtet:

Die Komponenten wurden in 10 mM Tris, 100 mM NaCl, pH 7,8 zu einer Konzentration von 6.67 μg/ml verdünnt. Pro Röhrchen wurden je 300 μl der Lösungen pipettiert und 20 h bei 22°C inkubiert. Die Lösungen wurden abgesaugt. Dann wurde jedes Röhrchen mit 300 μl 0,5% Bovines Serum Albumin, 2% Karion FP 2 h abgesättigt und nochmals abgesaugt. Anschließend wurden die Röhrchen in einem Vakuumtrockner getrocknet und bei 4°C gelagert.

### 3. Ni-NTA-Festphase

Es wurden Ni-NTA Mikrotiterplatten (Firma Qiagen, Hilden, Deutschland Art. Nr. 1006387) verwendet.

### 4. Assay zum Nachweis von reversibel gebundenen Antikörpern

### 4.1 Bindung der MACN-Antikörper-Konjugate

Die MACN-Antikörper-Konjugate wurden auf eine Konzentration von 1,5 μg/ml mit Verdünnungspuffer (s. Tabelle 2) eingestellt. Je 300 μl wurden in mehreren parallelen Ansätzen auf die entsprechende Festphase pipettiert und über Nacht bei Raumtemperatur inkubiert. Die Röhrchen wurden mit 5x1 ml, die Ni-NTA Mikrotiterplatte mit 5x 300 μl Waschpuffer (s. Tabelle 2) gewaschen.

In je einem der Ansätze wurde die Menge an gebundenem MACN-Antikörper-Konjugat bestimmt:

Die an den Röhrchen gebundene Menge an MACN-Antikörper-Konjugat wurde in einem Luminometer (Firma BERTHOLD, BAD WILDBAD, DEUTSCHLAND, LB952T; Basisreagenzien BRAHMS AG) vermessen. Die an der Mikrotiterplatte gebundene Menge an MACN-Antikörper-Konjugat wurde in einem Microplate Luminometer (Firma BERTHOLD, BAD WILDBAD, DEUTSCHLAND, MPL2; Basisreagenzien BRAHMS AG) vermessen.

### 4.2 Messung der unspezifischen Ablösung der MACN-Antikörper-Konjugate mit Vollblut

In weitere Ansätze der gebundenen MACN-Antikörper-Konjugate wurden je 300 μl Vollblut (s. Tabelle 2) pipettiert und 10 min inkubiert. Die Röhrchen wurden mit 5x1 ml, die Ni-NTA Mikrotiterplatte mit 5x 300 μl Waschpuffer (s. Tabelle 2) gewaschen. Die an den Röhrchen verbliebene Menge an MACN-Antikörper-Konjugat wurde in einem Luminometer (Firma BERTHOLD, BAD WILDBAD, DEUTSCHLAND, LB952T; Basisreagenzien BRAHMS AG) vermessen.

Die an der Mikrotiterplatte verbliebene Menge an MACN-Antikörper-Konjugat wurde in einem Microplate Luminometer (Firma BERTHOLD, BAD WILDBAD, DEUTSCHLAND, MPL2; Basisreagenzien BRAHMS AG) vermessen.

### 4.3 Messung der spezifischen Ablösung der MACN-Antikörper-Konjugate mit Ablösungspuffer

In weitere Ansätze der gebundenen MACN-Antikörper-Konjugate wurden je 300 μl Ablösungspuffer (s. Tabelle 2) pipettiert und 10 bzw. 30 min inkubiert. Die Röhrchen wurden mit 5x1 ml, die Ni-NTA Mikrotiterplatte mit 5 x 300 μl Waschpuffer gewaschen (s. Tabelle 2). Die an den Röhrchen verbliebene Menge an MACN-Antikörper-Konjugat wurde in einem Luminometer (Firma BERTHOLD, BAD WILDBAD, DEUTSCHLAND, LB952T; Basisreagenzien BRAHMS AG) vermessen.

Die an der Mikrotiterplatte verbliebene Menge an MACN-Antikörper-Konjugat wurde in einem Microplate Luminometer (Firma BERTHOLD, BAD WILDBAD, DEUTSCHLAND, MPL2; Basisreagenzien BRAHMS AG) vermessen.

Tabelle 2

| Festphase | MACN-Antikörper + | Verdünnungspuffer | Waschpuffer | Vollblut | Ablösungspuffer |
|---|---|---|---|---|---|
| Avidin | Biotin | PBS pH 7,4 0,5% BSA | 8 mM Tris pH 7,5 60 mM NaCl, 0,2% Tween 20 | EDTA-Vollblut | PBS pH 7,4, 0,5% BSA, 25 mM Biotin |
| Ni-NTA | His-tag Peptid | | | | PBS pH 7,4, 0,5% BSA, 250 mM Imidazol |
| Oligo-Lys BSA | Oligo-Asp Peptid | | | | PBS pH 7,4, 0,5% BSA, 0,6M KF, 0,01% Heparin |

(fortgesetzt)

| Festphase | MACN-Antikörper + | Verdünnungspuffer | Waschpuffer | Vollblut | Ablösungspuffer |
|---|---|---|---|---|---|
| FLAG-tag BSA | anti FLAG-tag M1 | 50 mM HEPES pH 7,2 150 mM NaCl, 0,02% Tween, 0,5% BSA, 5 mM $CaCl_2$ | 50 mM HEPES pH 7,2 150 mM NaCl, 0,02% Tween, 5 mM $CaCl_2$ | Heparin-Vollblut | 50 mM HEPES pH 7,2 150 mM NaCl, 0,02% Tween, 0,5% BSA, 50 mM EDTA |
| ProtC-tag BSA | anti ProtC-tag HPC4 | | | | |

**Weitergehende Untersuchungen zum Oligo-Asp/Oligo-Lys-System**

**Ablösungsagentien**

Wie oben beschrieben, wurde eine effiziente Auflösung der Bindung durch Zugabe von 0,01 % Heparin erreicht (s. Tabelle 1 und zugehörige Beschreibung). Als alternative Ablösungsagentien wurden auch NaCl und KF untersucht. Eine mit 0,01% Heparin vergleichbare Wirkung auf die Auflösung der Bindung wurde mit NaCl- bzw. KF-Konzentrationen >0.8 M erreicht. Es sind zahlreiche andere Variationen in der Pufferkomposition denkbar, die zu Auflösung der Bindung führen (andere Salze von geringem Molekulargewicht, andere geladene Oligo- oder Polymere, anderer pH-Wert). All diese Variationen sind im Sinne dieser Erfindung als Ablösungsagentien zu verstehen. Als besonders bevorzugt sind jedoch solche Variationen anzusehen, die einerseits effizient die Bindung des Oligo-Asp/Oligo-Lys-Systems destabilisieren, andererseits aber die Bindung zwischen Antikörper und Analyten möglichst unbeeinträchtigt lassen. Heparin in einer Konzentration um 0.01% stellt eine solche bevorzugte Variante dar, da bekanntermaßen viele Immunoassays als Probenmatrix Heparinplasma verwenden können und die Konzentration von Heparin in diesem Plasma typischerweise im Bereich von 0.01% liegt.

**Spezifität der Bindung des Analyten an die Festphase**

Gibt man eine biologische Probe zu einer Festphase, die beschichtet ist mit Oligo-Lys-BSA/Oligo-Asp-Analyt-Binder, so könnte man befürchten, dass der Analyt von Interesse aus der Probe nicht nur an den Analyt-spezifischen Antikörper bindet, sondern möglicherweise auch unspezifisch über ionische Wechselwirkung an eventuell freie Lysin Anteile der Festphase. Wäre dies der Fall, wäre die Richtigkeit in der nachfolgenden Analytdetektion (nach dem Ablöseschritt) beeinträchtigt. Ob und inwieweit derartige unspezifische Analytbindungen erfolgen können, wurde untersucht, indem verschiedene synthetische Peptide, welche weitgehend identisch mit bekannten natürlichen Analyten sind und diese daher repräsentieren, chemilumineszenz-markiert wurden, in eine Plasmamatrix verdünnt wurden und in Röhrchen inkubiert wurden, die mit Oligo-Lys-BSA beschichtet und daran ein Oligo-Asp-anti-PCT Antikörper gebunden war. Der detektierte Anteil an unspezifisch gebundenen Peptiden lag immer unter 7% des angebotenen Peptids. Somit stellt unspezifische Analytbindung an die Festphase kein Problem dar.

Im Einzelnen wurde der Versuch wie folgt durchgeführt:

**1. Herstellung von MACN-chemilumineszenz-markierten Peptiden**

Folgende Peptide (alle von Firma JPT GmbH, Berlin, Deutschland) wurden mit MACN markiert:

"PPL41" (Aminosäuresequenz PEVPPWTGEVSPAQRDGGALGGGGRGPWDSSDRSALLKSKL, (SEQ ID NO. 6) abgeleitet von NT-proANP), "PSW44" (Aminosäuresequenz SSEEHLRQTRSETMRNSVKSSFHDPKLKGKPSRERYVTHNRAHW, (SEQ ID NO. 7), abgeleitet von proEndothelin), "PAY33" (Aminosäuresequenz ATQLDGP AGALLLRLVQLAGAPEPFEPAQPDAY, (SEQ ID NO. 8) abgeleitet von Copeptin), "Peptid 45-92" (Aminosäuresequenz ELRMSSSYPTGLADVKAGPAQTLIRPQDMKGASRSPEDSSPDAARIRV, (SEQ ID NO. 9) abgeleitet von proAdrenomedullin).

Zur Chemilumineszenzmarkierung wurden die Peptide im molaren Verhältnis von 1:1 mit MACN-Akridinium-NHS-Ester (Firma InVent GmbH, Hennigsdorf, Deutschland) 1 Stunde bei Raumtemperatur markiert. Anschließend wurde die Reaktion mit 1M Tris pH 7,8 10 min gestoppt.

Zur Abtrennung von nicht eingebauten MACN wurden die Markierungsansätze über eine reversed phase C18-Säule (μBondapak, Waters WAT027342, Nr. 0202352581) gereinigt. Die Proben wurden aufgetragen und bei einer Flussrate von 1,0 ml/min mittels eines ansteigenden Wasser/Acetonitril- (+0,1% TFA)-Gradienten chromatographiert. Mit einem Durchflussphotometer wurden die Wellenlängen 214nm, 280nm und 368 nm gemessen und die Peaks detektiert.

Die markierten Peptide wurden portioniert bei -20°C gelagert.

**1.1. Herstellung BSA+Oligo-Lys-Konjugat: siehe oben**

**1.2. Herstellung anti-Calcitonin Schaf Antikörper+Oligo-Asp-Konjugat**

Zur Kopplung des Oligo-Asp Peptids "D14C" (Aminosäuresequenz DDDDDDDDDDDDDC, (SEQ ID NO. 2), (Firma JPT GmbH, Berlin, Deutschland, M=1731,43) wurde ein polyklonaler anti-Calcitonin Schaf Antikörper ("anti-PCT Antikörper 1") mit SMCC im Verhältnis 1:25 aktiviert (siehe "Instructions SMCC and Sulfo-SMCC", Pierce).

3 mg Antikörper (in 4,5 ml PBS, 10 mM EDTA, pH 8) wurden mit 5,0 μl 100 mM SMCC (frisch gelöst in DMSO) versetzt und 30 min bei Raumtemperatur inkubiert. Das Produkt wurde über zwei NAP25-Säulen gereinigt (Laufmittel: PBS, 10 mM EDTA, pH 8) und der Proteingehalt photometrisch bestimmt.

Anschließend erfolgte sofort die Konjugation mit dem Oligo-Asp Peptid (gelöst in dest. Wasser) im molaren Verhältnis von 1:100. Nach Inkubation von 60 min bei Raumtemperatur wurde die Reaktion mit 100 μl 100 mM Cystein 10 min gestoppt, nicht konjugiertes Peptid über NAP25-Säulen abgetrennt (Laufmittel: PBS, pH 7,4) und der Proteingehalt photometrisch bestimmt.

Das anti-Calcitonin Schaf Antikörper+Oligo-Asp-Konjugat wurde aliquotiert bei -20°C gelagert.

**2. Kopplung der Konjugate auf Röhrchen**

**2.1. Kopplung: BSA+Oligo-Lys-Konjugat**

Bestrahlte Sternröhrchen (Firma Greiner, Frickenhausen, Deutschland) wurden mit BSA-Oligo-Lys-Konjugat wie folgt beschichtet:

Das Konjugat wurde in 10 mM Tris, 100 mM NaCl, pH 7,8 zu einer Konzentration von 6.67 μg/ml verdünnt. In jedes Röhrchen wurden 300 μl dieser Lösung pipettiert und 20 h bei 22°C inkubiert. Die Lösung wurde abgesaugt. Dann wurde jedes Röhrchen mit 300 μl 0,5% Bovines Serum Albumin, 2% Karion FP 2 h abgesättigt und nochmals abgesaugt. Anschließend erfolgte die Bindung des anti-Calcitonin Schaf Antikörper+Oligo-Asp-Konjugates:

**2.2. Bindung: anti-Calcitonin Schaf Antikörper+Oligo-Asp-Konjugat**

Das Konjugat wurde in PBS, 0,5% proteasefreies BSA pH 7,4 zu einer Konzentration von 667 ng/ml verdünnt. In jedes Röhrchen wurden 300 μl dieser Lösung pipettiert und 1 Stunde bei 22°C inkubiert. Die Lösung wurde abgesaugt. Dann wurde jedes Röhrchen mit 300 μl 0,5% Bovines Serum Albumin, 2% Karion FP 2 h nochmals abgesättigt und danach abgesaugt.

Anschließend wurden die Röhrchen in einem Vakuumtrockner getrocknet und bei 4°C gelagert.

**3. Bestimmung der unspezifischen Bindung:**

Die MACN-Peptide wurden in einer Plasmamatrix verdünnt. Je 300 μl wurden in die mit dem Oligo-Lys-BSA und dem Oligo-Asp-anti-PCT Antikörper beschichteten Röhrchen pipettiert und 30 min bei Raumtemperatur inkubiert. Die Röhrchen wurden mit 3x 1 ml PBS pH 7,4, 0,5% proteasefreies BSA gewaschen. Die an den Röhrchen gebundene Menge sowie die eingesetzte Totalaktivität an MACN-Peptid wurde in einem Luminometer (Firma BERTHOLD, BAD WILDBAD, DEUTSCHLAND, LB952T; Basisreagenzien BRAHMS AG) gemessen. Die prozentuale unspezifische Bindung bezogen auf die Totalaktivität betrug:

| | |
|---|---|
| PSW44 | 3,0% |
| PPL41 | 3,1% |
| PAY33 | 6,5% |
| Peptid 45-92 | 2,9% |

**Vergleich am Beispiel PCT: Klassische Immunchromatographie aus Vollblut vs. Immunextraktion von PCT aus Vollblut, Ablösung und Analytdetektion mittels Immunchromatographie oder der TRACE Technologie**

PCT (Procalcitonin) wurde als relevanter Modellanalyt ausgewählt. Es handelt sich um einen Marker für bakterielle Infektionen, insbesondere schwere Infektionen, die zu einer systemischen Inflammationsreaktion des Wirts führen (Sepsis) (21). Sandwich Immunoassays zur Detektion von PCT verwenden Antikörper gegen den Calcitonin- bzw. Katacalcin-Anteil des Peptids (22), (23).

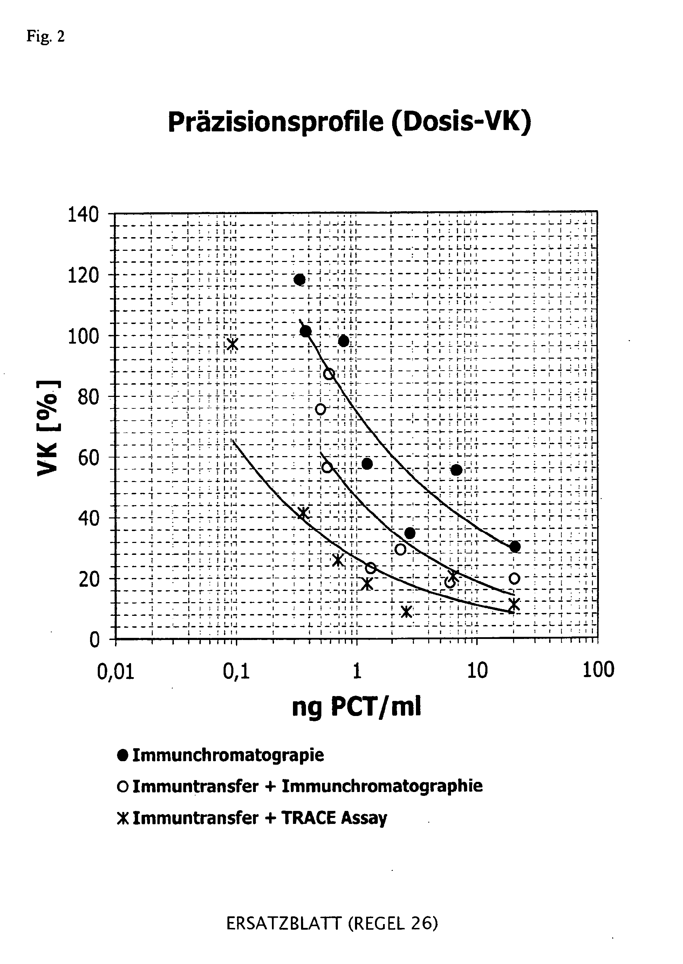

Die Immunchromatographie stellt die in der heutigen Routineanwendung gebräuchlichste Schnelltest-Methodik zur immunologischen Detektion von Analyten aus Vollblut dar und kann daher als "klassische Immunchromatographie" bezeichnet werden. Diese Methodik wurde daher hier als Referenzmethode gewählt, um Vorteile des erfindungsgemäßen Prinzips der Immunextraktion und Ablösung ("Immuntransfer") mit angeschlossener Analytdetektion darzulegen. Es wurden zwei verschiedene Detektionsmethoden untersucht: Einerseits die Immunchromatographie, andererseits die TRACE (Time resolved amplified cryptate emission) Technologie. Mit allen drei Methoden wurden PCT Konzentrationen aus PCT enthaltenden Vollblutproben in jeweils 10-fach-Bestimmung ermittelt. In Figur 2 sind die resultierenden Präzisionsprofile der Methoden gegenübergestellt. Es ist deutlich zu erkennen, dass beide Methoden, die Gegenstand dieser Erfindung sind, also Immuntransfer in Verbindung mit einer Detektionsmethode (sei es Immunchromatographie oder TRACE Technologie), wesentlich präzisere und sensitivere Messergebnisse liefern als die Referenzmethode (klassische Immunchromatographie direkt aus Vollblut). Die Einzelheiten zur Durchführung der Experimente werden im Folgenden erläutert. Unter A ist als erfindungsgemäßes Verfahren Immuntransfer mit angeschlossener Analytdetektion mittels TRACE Technologie beschrieben; unter B ist als erfindungsgemäßes Verfahren Immuntransfer mit angeschlossener Analytdetektion mittels Immunchromatographie beschrieben; unter C ist als Referenzmethode die klassische Immunchromatographie beschrieben. Ein Vergleich der Präzisionsprofile der Varianten A, B und C ist in Figur 2 dargestellt.

**A. Immunextraktion von PCT aus Vollblut und Analytdetektion mittels der TRACE Technologie**

Als Festphase wurden Mikrotiterplatten aus hochbindendem Polystyrol verwendet, die mit Oligo-Lys-BSA beschichtet waren, woran ein erster anti-PCT-Antikörper gebunden war, der vorher mit Oligo-Asp und Kryptat derivatisiert worden war. Darauf wurde die PCT enthaltende Vollblutprobe kurz inkubiert und das PCT aus der Probe auf die Festphase immunextrahiert. Nach Waschen der Festphase wurde der Ablösungspuffer zugegeben und kurz inkubiert. Ein Aliquot wurde in eine andere, niedrig bindende schwarze Mikrotiterplatte überführt und mit einer Lösung gemischt, die einen zweiten, mit Cyanin markierten anti-PCT Antikörper enthielt. Nach kurzer Inkubation wurde die Sandwichbildung nach Anregung mit Laser-Licht mittels TRACE Technologie detektiert.

Im Einzelnen wurde der Versuch wie folgt durchgeführt:

**A.1. Herstellung der Konjugate für die Festphase**

**A.1.1. Herstellung BSA+Oligo-Lys-Konjugat: s. oben**

**A.1.2. Herstellung anti-Calcitonin Schaf Antikörper-Kryptat MonoMP+Oligo-Asp-Konjugat**

Für die Markierung mit Kryptat Monophosphat ("KMonoMP", Firma Cezanne, Nimes, Frankreich,) wurde ein polyklonaler anti-Calcitonin-Schaf-Antikörper ("anti-PCT Antikörper 1") mit SPDP (N-succinimidyl 3-(2-pyridyldithio) propionate, Firma Pierce, Rockford, IL, USA, Art. Nr. 21857) im molaren Verhältnis von 1:4 aktiviert (siehe "Instructions SPDP Reagents", Pierce, Rockford, IL, USA).

5 mg Antikörper (gelöst in 1,5 ml 100 mM Natriumphosphat, 5 mM EDTA, pH 6,9) wurden mit 41,6 μl 3,2 mM SPDP (frisch gelöst in Ethanol) versetzt und 30 min bei Raumtemperatur inkubiert.

Der aktivierte Antikörper wurde mit 81,2 μl 200 mM DTT (Dithiothreitol, gelöst in 100 mM Natriumphosphat, 5 mM EDTA, pH 6,9) 15 min bei Raumtemperatur reduziert. Das Produkt wurde über zwei NAP10-Säulen gereinigt (Laufmittel: 100 mM Natriumphosphat, 5 mM EDTA, pH 6,9) und der Proteingehalt photometrisch bestimmt.

Anschließend erfolgte sofort die Konjugation des Eluats mit lyophilisiertem KMonoMP im molaren Verhältnis von 1:8. Nach Inkubation von 20 h bei 4°C wurde das Produkt nochmals über drei NAP10-Säulen gereinigt (Laufmittel: PBS, 10 mM EDTA pH 8) und der Proteingehalt photometrisch bestimmt.

Zur Kopplung des Oligo-Asp Peptids "D14C" (Aminosäuresequenz DDDDDDDDDDDDDC, (SEQ ID NO. 2), Firma JPT GmbH, Berlin, Deutschland, M=1731,43) wurde das Produkt mit SMCC im Verhältnis 1:25 aktiviert (siehe "Instructions SMCC and Sulfo-SMCC", Pierce, Rockford, IL, USA): 3 mg Produkt (eluiert mit 4,5 ml PBS, 10 mM EDTA, pH 8) wurden mit 5,0 μl 100 mM SMCC (frisch gelöst in DMSO) versetzt und 30 min bei Raumtemperatur inkubiert. Das Produkt wurde über zwei NAP25-Säulen gereinigt (Laufmittel: PBS, 10 mM EDTA, pH 8) und der Proteingehalt wurde photometrisch bestimmt.

Anschließend erfolgte sofort die Konjugation mit dem Oligo-Asp Peptid (gelöst in dest. Wasser) im molaren Verhältnis von 1:100. Nach Inkubation von 60 min bei Raumtemperatur wurde die Reaktion mit 100 $\mu$l 100 mM Cystein 10 min gestoppt, nicht konjugiertes Peptid über NAP25-Säulen abgetrennt (Laufmittel: PBS, pH 7,4) und der Proteingehalt wurde photometrisch bestimmt.

Das anti-Calcitonin Schaf Antikörper-K MonoMP+Oligo-Asp-Konjugat wurde aliquotiert bei - 20°C gelagert.

**A.2. Kopplung der Konjugate auf Mikrotiterplatten**

**A.2.1. Kopplung: BSA+Oligo-Lys-Konjugat**

Bestrahlte Mikrotiterplatten (Firma Greiner, Frickenhausen, Deutschland) wurden mit BSA-Oligo-Lys-Konjugat wie folgt beschichtet:

Das Konjugat wurde in 10 mM Tris, 100 mM NaCl, pH 7,8 zu einer Konzentration von 6.67 $\mu$g/ml verdünnt. In jede Kavität wurden 300 $\mu$l dieser Lösung pipettiert und 20 h bei 22°C inkubiert. Die Lösung wurde abgesaugt. Dann wurde jede Kavität mit 300 $\mu$l 0,5% Bovines Serum Albumin, 2% Karion FP 2 h abgesättigt und nochmals abgesaugt. Anschließend erfolgte die Bindung des anti-Calcitonin Schaf Antikörper-Kryptat MonoMP+Oligo-Asp-Konjugates:

**A.2.2. Bindung: anti-Calcitonin Schaf Antikörper-Kryptat MonoMP+Oligo-Asp-Konjugat**

Das Konjugat wurde in PBS, 0,5% proteasefreies BSA pH 7,4 zu einer Konzentration von 667 ng/ml verdünnt. In jede Kavität wurden 300 $\mu$l dieser Lösung pipettiert und 1 Stunde bei 22°C inkubiert. Die Lösung wurde abgesaugt. Dann wurde jede Kavität mit 300 $\mu$l 0,5% Bovines Serum Albumin, 2% Karion FP 2 h abgesättigt und nochmals abgesaugt.

Anschließend wurden die Mikrotiterplatten in einem Vakuumtrockner getrocknet und bei 4°C gelagert.

**A.3. Herstellung eines anti-Katacalein Maus Antikörper-Cy5-Konjugats für die Flüssigphase**

Ein monoklonaler anti-Katacalcin Antikörper ("anti-PCT Anitkörper 2") wurde in Anwesenheit von 45% (2-Hydroxyl-propyl)-ß-Cyclodextrin (Firma Aldrich, Seelze, Deutschland, Art. Nr.

33.260-7) mit Cy5-NHS (Firma Amersham, Art. Nr. PA15100) im molaren Verhältnis von 1:20 markiert (siehe Product Booklet "Amersham CyDye mono-reactive NHS Esters").

213 $\mu$l Antikörper (c=10 mg/ml in PBS pH 7,4) wurden mit 750 $\mu$l 60% Cyclodextrin (gelöst in 100 mM Carbonatpuffer pH 9) gemischt und mit 37,3 $\mu$l 7,7 mM Cy5-NHS (gelöst in DMSO) versetzt. Nach Inkubation von einer Stunde bei Raumtemperatur wurde der Ansatz mit 50 $\mu$l 50 mM Glycin 10 min gestoppt und über eine NAP10-Säule (Laufmittel: PBS, pH 7,4) vorgereinigt.

Zur Abtrennung letzter Reste nicht an Antikörper gebundenen Cy5-NHS wurde eine Gelfiltrations-HPLC durchgeführt (Säule: Bio-Sil Sec 125, Firma Biorad, München, Deutschland). Die Probe wurde aufgetragen und bei einer Flussrate von 1 ml/min mit PBS, pH 7,4 chromatographiert. Mit einem Durchflussphotometer wurden die Wellenlängen 280 nm und 648 nm gemessen. Das Absorbtionsverhältnis 648 nm/280 nm als Maß für den Markierungsgrad des Antikörpers betrug am Peak 1,48. Die Antikörper enthaltenden Fraktionen (Retentionszeit 8-10 min) wurden gepoolt.

Das anti-Katacalcin Maus Antikörper-Cy5-Konjugat wurde portioniert und bei -20° C gelagert.

**A.4. Immuntransfer und TRACE Assay**

Der im Folgenden beschriebene Assay wurde für jede Probe in 10-fach Bestimmung durchgeführt. In die Kavitäten der mit BSA+Oligo-Lys und anti-Calcitonin Schaf Antikörper-Kryptat MonoMP+D14C-Konjugat beschichteten Mikrotiterplatten wurden 300 $\mu$l EDTA-Vollblut Proben, aufgestockt mit rekombinantem PCX (InVivo GmbH, Hennigsdorf, Deutschland), pipettiert und 15 min bei Raumtemperatur inkubiert (die PCT Konzentrationen der Proben wurden durch Messung mit dem Assay BRAHMS PCT LIA sensitiv (BRAHMS AG, Hennigsdorf, Deutschland) aus Plasma bestimmt). Danach wurde 2x mit 300 $\mu$l PBS, pH 7,4, dann 2x mit 300 $\mu$l Waschlösung (8 mM Tris, 60 mM NaCl, 0,2% Tween 20, pH 7,5) gewaschen. Anschließend wurde mit 300 $\mu$l Ablösungspuffer (PBS pH 7,4, 0,6M KF, 0,5% BSA, 0,01% Heparin) 10 min inkubiert. Von dem abgelösten Immunkomplex wurden 100 $\mu$l in für die TRACE-Messung geeignete Mikrotiterplatten (Costar Half Area Plates, 96 Well, schwarz, Polystyrol, Firma Sigma/Aldrich, Seelze, Deutschland, Cat. No. CLS 3694-100EA) überführt und mit 50 $\mu$l anti-Katacalcin Maus Antikörper-Cy5-NHS Konjugat (c=20 $\mu$g/ml in PBS pH 7,4, 0,6M KF, 0,5% BSA, 0,01% Heparin) 19 min bei Raumtemperatur inkubiert. Die Messung der TRACE-Signale (620 nm und 665 nm) erfolgte mittels dem High-Performance Time-Resolved Fluorescence Microtiterplate Reader RUBYstar (Firma BMG LABTECH GmbH, Offenburg, Deutschland).

Folgende Geräteeinstellungen wurden dabei am RUBYstar vorgenommen:

| Number of flashes | 20 |
| Integration delay [$\mu$s] | 50 |
| Integration time [$\mu$s] | 400 |
| Interval time [$\mu$s] | 10 |
| Number of intervals | 45 |
| Ratio multipHcator | 10000 |

Für jede Probe wurde aus dem Ratio $OD_{665nm}/OD_{620nm}$ Delta F wie folgt berechnet:

$$\text{Delta F} = (\text{Ratio}_{pos} - \text{Ratio}_{neg}) / \text{Ratio}_{neg}$$

Dabei meint $Ratio_{neg}$ das Ratio einer PCT-negativen Probe (also PCT nicht enthaltenden bzw. nicht detektierbaren Probe) und $Ratio_{pos}$ das Ratio einer zu testenden Probe (also einer potentiell PCT enthaltenden Probe). Aus den 10-fach Bestimmungen der einzelnen Proben wurde der jeweilige Mittelwert gebildet. Diese Werte dienten als Standardkonzentrationen, an denen die Konzentrationen aller einzelnen Proben unter Verwendung der Software MultiCalc (Spline Fit) ermittelt wurden. Zu jeder 1 0-fach Bestimmung wurde der Variationskoeffizient bestimmt.

B. Immunextraktion von PCT aus Vollblut und Analytdetektion mittels Immunchromatographie

Als Festphase wurden Polystyrolröhrchen verwendet, die mit Oligo-Lys-BSA beschichtet waren, woran ein erster anti-PCT-Antikörper gebunden war, der vorher mit Oligo-Asp und Biotin derivatisiert worden war. Darauf wurde PCT enthaltende Vollblutprobe kurz inkubiert und dabei PCT aus der Probe auf die Festphase immunextrahiert. Nach Waschen der Festphase wurde Ablösungspuffer zugegeben, der einen zweiten, mit kolloidalem Gold markierten anti-PCT Antikörper enthielt, und kurz inkubiert. Die Lösung wurde schließlich auf einem Teststreifen immunchromatographiert, auf den als Testbande Streptavidin aufgesprüht war. An das Streptavidin bindet der biotinylierte erste anti-PCT Antikörper und damit auch daran gebundenes PCT und der wiederum daran gebundene zweite Gold-markierte anti-PCT Antikörper. Die Detektion des Labels erfolgte reflektrometrisch.

Im Einzelnen wurde der Versuch wie folgt durchgeführt:

**B.1. Herstellung der Konjugate für die Festphase**

**B.1.1. Herstellung BSA+Oligo-Lys-Konjugat: siehe oben**

**B.1.2. Herstellung anti-Calcitonin Schaf Antikörper biotinyliert+Oligo-Asp-Konjugat**

Ein polyklonaler anti-Calcitonin Schaf Antikörper ("anti-PCT Antikörper 1") wurde mit EZ-Link Sulfo-NHS-LC-LC-Biotin (Firma Pierce, Rockford, IL, USA, Art. Nr. 21338) im molaren Verhältnis von 1:10 versetzt (siehe "Instructions EZ-Link Sulfo-NHS-LC-LC-Biotin", Pierce, Rockford, IL, USA): 1,6 mg Antikörper (in PBS pH 7,4) wurden mit 10 $\mu$l 10 mM EZ-Link Sulfo-NHS-LC-LC-Biotin (frisch gelöst in dest. Wasser) versetzt und 30 min bei Raumtemperatur inkubiert. Das Produkt wurde über eine NAP5-Säule gereinigt (Laufmittel: PBS, 10 mM EDTA pH 8,0) und der Proteingehalt photometrisch bestimmt.

Zur Kopplung des Oligo-Asp Peptids "D14C" (Aminosäuresequenz DDDDDDDDDDDDDC, (SEQ ID NO. 2), (Firma JPT GmbH, Berlin, Deutschland, M=1731,43) wurde das Produkt mit SMCC im Verhältnis 1:50 aktiviert (siehe "Instructions SMCC and Sulfo-SMCC", Pierce, Rockford, IL, USA).

1,3 mg Produkt (eluiert mit 1,0 ml PBS, 10 mM EDTA, pH 8) wurden mit 4,33 $\mu$l 100 mM SMCC (frisch gelöst in DMSO) versetzt und 30 min bei Raumtemperatur inkubiert. Das Produkt wurde über eine NAP10-Säule gereinigt (Laufmittel: PBS, 10 mM EDTA, pH 8) und der Proteingehalt photometrisch bestimmt.

Anschließend erfolgte sofort die Konjugation mit dem Oligo-Asp Peptid (gelöst in dest. Wasser) im molaren Verhältnis von 1:100. Nach Inkubation von 60 min bei Raumtemperatur wurde die Reaktion mit 100 $\mu$l 100 mM Cystein 10 min gestoppt, nicht konjugiertes Peptid über eine NAP25-Säule abgetrennt (Laufmittel: PBS, pH 7,4) und der Proteingehalt photometrisch bestimmt.

Das anti-Calcitonin Schaf Antikörper biotinyliert+Oligo-Asp-Konjugat wurde aliquotiert bei -20°C gelagert.

**B.2. Kopplung des Konjugats auf Röhrchen**

**B.2.1. Kopplung: BSA+Oligo-Lys-Konjugat**

Bestrahlte Sternröhrchen (Firma Greiner, Frickenhausen, Deutschland) wurden mit BSA-Oligo-Lys-Konjugat wie folgt beschichtet:

Das Konjugat wurde in 10 mM Tris, 100 mM NaCl, pH 7,8 zu einer Konzentration von 6.67 $\mu$g/ml verdünnt. In jedes Röhrchen wurden 300 $\mu$l dieser Lösung pipettiert und 20 h bei 22°C inkubiert. Die Lösung wurde abgesaugt. Dann wurde jedes Röhrchen mit 300 $\mu$l 0,5% Bovines Serum Albumin, 2% Karion FP 2 h abgesättigt und nochmals abgesaugt: Anschließend erfolgte die Bindung des anti-Calcitonin Schaf Antikörper biotinyliert+Oligo-Asp-Konjugates:

**B.2.2. Bindung: anti-Calcitonin Schaf Antikörper biotinyliert+Oligo-Asp-Konjugat**

Das Konjugat wurde in PBS, 0,5% proteasefreies BSA pH 7,4 zu einer Konzentration von 667 ng/ml verdünnt. In jedes Röhrchen wurden 300 $\mu$l dieser Lösung pipettiert und 1 Stunde bei 22°C inkubiert. Die Lösung wurde abgesaugt. Dann wurde jedes Röhrchen mit 300 $\mu$l 0,5% Bovines Serum Albumin, 2% Karion FP 2 h nochmals abgesättigt und danach abgesaugt.

Anschließend wurden die Röhrchen in einem Vakuumtrockner getrocknet und bei 4°C gelagert.

**B.3. Herstellung von Gold-markiertem anti-Katacalcin Antikörper:**

Diese Komponente wurde von Firma 8sens.biognostic GmbH, Berlin, Deutschland, erworben. Monoklonaler anti-Katacalcinin Antikörper wurde an kolloidales Gold (Firma 8sens.biognostic GmbH, Berlin, Deutschland; nach Frens: Frens, G., Nature Physical Science, 1973, 241 20-22) wie bei Hermanson beschrieben gekoppelt (http://www.amazon.deBioconjugate-TechniquesGreg-T-Hermanson/dp/0123423368).

**B.4. Herstellung der Teststreifen:**

Diese Komponente wurde von Firma 8sens.biognostic GmbH, Berlin, Deutschland, erworben. Es wurden Nitrocellulose-Membranen von MDI (Advanced Microdevices, Ambala, Indien) mit 4 $\mu$g/cm Streptavidin (Pierce, Rockford, IL, USA) (Test-Linie) sowie mit 2,5 $\mu$g/cm anti-Maus IgG Antikörper (Scantibodies, Santee, CA, USA) (Kontroll-Line) beschichtet. Zum Aufbringen der Fänger-Moleküle wurde ein Mikrodispenser der Firma Biodot, Irvine, CA, USA, eingesetzt. Anschließend erfolgte die Trocknung der Membran über Nacht bei 50°C. Als Saugpad kam AP22 der Firma Millipore, Billerica, MA, USA zum Einsatz.

**B.5. Immuntransfer und Immunchromatographie:**

Der im Folgenden beschriebene Assay wurde für jede Probe in 10-fach Bestimmung durchgeführt. In die mit BSA+Oligo-Lys und anti-Calcitonin Schaf Antikörper biotinyliert+D14C-Konjugat beschichteten Röhrchen wurden 300 $\mu$l EDTA-Vollblut Proben, aufgestockt mit rekombinantem PCT (InVivo GmbH, Hennigsdorf, Deutschland) pipettiert und 15 min bei Raumtemperatur inkubiert (die PCT Konzentrationen der Proben wurden durch Messung mit dem Assay BRAHMS PCT LIA sensitiv (BRAHMS AG, Hennigsdorf, Deutschland) aus Plasma bestimmt). Danach wurde 2x mit 300 $\mu$l PBS, pH 7,4, dann 2x mit 300 $\mu$l Waschlösung (8 mM Tris, 60 mM NaCl, 0,2% Tween 20, pH 7,5) gewaschen. Anschließend wurde mit 150 $\mu$l Ablösungspuffer (PBS pH 7,4, 5% BSA, 0,5% Tween 20, 0,01% Heparin), der auch den Gold-markierten anti-Katacalcin monoklonalen Antikörper enthielt, unter Schütteln für 10 min inkubiert. Von dem abgelösten Immunkomplex wurden 150 $\mu$l in Kavitäten von low binding Mikrotiterplatten überführt. Anschließend erfolgte unmittelbar die Immunchromatographie mit den Teststreifen, indem diese in die Kavitäten gesteckt wurden. Nach 30 min wurde die Färbung der Testbande mit einem Reflektrometer der Firma LRE Medical GmbH, München, Deutschland, bestimmt.

Aus den 10-fach Bestimmungen der einzelnen Proben wurde der jeweilige Mittelwert gebildet. Diese Werte dienten als Standardkonzentrationen, an denen die Konzentrationen aller einzelnen Proben unter Verwendung der Software MultiCalc (Spline Fit) ermittelt wurden. Zu jeder 10- fach Bestimmung wurde der Variationskoeffizient bestimmt.

**C. Detektion von PCT aus Vollblut mittels klassischer Immunchromatographie**

Es wurden Teststreifen hergestellt, die an der Probenauftragszone mit einer Membran versehen waren, die geeignet war, Blutzellen aus einer aufzutragenden Vollblutprobe zurückzuhalten. Zwischen dieser Membran und dem Teststreifen war ein poröses Kissen angebracht ("Proben-Pad"), das eingetrocknet einerseits einen ersten, biotinylierten anti-PCT Antikörper enthielt, andererseits einen zweiten, mit kolloidalem Gold markierten anti-PCT Antikörper. Als Testbande war auf den Streifen Streptavidin aufgesprüht. Die Teststreifen waren in Plastik-Kassetten eingefügt. Die PCT enthaltende Vollblutprobe wurde immunchromatographiert. An das Streptavidin bindet der biotinylierte erste anti-PCT Antikörper und damit auch daran gebundenes PCT und der wiederum daran gebundene zweite, Gold-markierte anti-PCT Antikörper. Die Detektion des Labels erfolgte reflektrometrisch.

Im Einzelnen wurde der Versuch wie folgt durchgeführt:

**C.1. Herstellung der Konjugate für die Festphase**

**C.1.1. Herstellung BSA+Oligo-Lys-Konjugat: siehe oben**

**C.1.2. Herstellung anti-Calcitonin Schaf Antikörper biotinyliert+Oligo-Asp-Konjugat: siehe oben**

**C.2. Herstellung von Gold-markiertem anti-Katacalcin Antikörper: siehe oben**

**C.3. Herstellung der Teststreifen:**

Diese Komponente wurde von Firma 8sens.biognostic GmbH, Berlin, Deutschland, erworben. Es wurden Nitrocellulose-Membranen von MDI (Advanced Microdevices, Ambala, Indien) mit 4 $\mu$g/cm Streptavidin (Pierce, Rockford, IL, USA) (Test-Linie) sowie mit 2,5 $\mu$g/cm anti-Maus IgG Antikörper (Scantibodies, Santee, CA, USA) (Kontroll-Line) beschichtet. Zum Aufbringen der Fänger-Moleküle wurde ein Mikrodispenser der Firma Biodot, Irvine, CA, USA, eingesetzt. Anschließend erfolgte die Trocknung der Membran über Nacht bei 50°C. Als Saugpad kam AP22 der Firma Millipore, Billerica, MA, USA zum Einsatz. Für die Herstellung der Vollblut-Tests wurde ein Vollblut setup der Firma 8sens.biognostic verwendet. Im Probenauftragsbereich wurden auf 2 getrennten Pads zum einen der an kolloidales Gold gekoppelte anti-Katacalcin Antikörper und zum anderen der biotinylierte+Oligo-Asp-derivatisierte anti-Calcitonin Antikörper immobilisiert. Als Kassette wurde ein Standard Housing der Firma 8sens.biognostic genutzt.

**C.4. Klassische Immunchromatographie**

Die im Folgenden beschriebene Immunchromatographie wurde für jede Probe in 10-fach Bestimmung durchgeführt. Auf die Teststreifen wurden 120 $\mu$l EDTA-Vollblut Proben, aufgestockt mit rekombinantem PCT (InVivo GmbH, Hennigsdorf, Deutschland) pipettiert (die PCT Konzentrationen der Proben wurden durch Messung mit dem Assay BRAHMS PCT LIA sensitiv (BRAHMS AG, Hennigsdorf, Deutschland) aus Plasma bestimmt). Nach 30 min wurde die Färbung der Testbande mit einem Reflektrometer der Firma LRE Medical GmbH, München, Deutschland, bestimmt.

Aus den 10-fachen Bestimmungen der einzelnen Proben wurde der jeweilige Mittelwert gebildet. Diese Werte dienten als Standardkonzentrationen, an denen die Konzentrationen aller einzelnen Proben unter Verwendung der Software MultiCalc (Spline Fit) ermittelt wurden. Zu jeder 10-flach Bestimmung wurde der Variationskoeffizient bestimmt.

**D. Immunextraktion von MR-proADM aus Vollblut und Analytdetektion mittels Immunchromatographie**

Als weiteres Beispiel für das Verfahren der Immunextraktion aus Vollblut und Analytdetektion mittels Immunchromatographie wurde ein Testsystem für den Analyten MR-proADM (mittregionales Pro-Adrenomedullin) entwickelt. MR-proADM ist ein stabiles im Blut zirkulierendes Peptid, das aus der proteolytischen Prozessierung des Vorläufers (precursors) von Adrenomedullin hervorgeht (Struck J et al: Peptides, 2004 Aug;25(8): 1369-72.). Adrenomedullin ist einer der am stärksten bekannten endogenen Vasodilatoren, das an der Regulation zahlreicher biologischer Prozesse beteiligt ist, insbesondere an der Regulation des kardiovaskulären Systems (Beltowski J et al.: Pol J Pharmacol. 2004 Jan-Feb;56(1):5-27.). Die Messung von MR-proADM hat sich als Surrogatmarker für das nicht zuverlässig messbare reife Adrenomedullin im Rahmen der Diagnose- und Prognosestellung verschiedener Krankheitszustände als nützlich erwiesen. Dazu zählen u.a.: COPD (Stolz D et al.: Chest. 2008 May 19. [Epub ahead ofprint]), Pneumonie (Christ-Crain M et al.: Crit Care. 2006;10(3):R96.), Sepsis (Christ-Crain M et al.: Crit Care. 2005;9(6):R816-24.), Herzinfarkt (Khan SQ et al.: J Am Coli Cardiol. 2007 Apr 10;49(14): 1525-32.), Herzinsuffizienz (Gegenhuber A et al.: J Card Fail. 2007 Feb;13(1):42- 9.).

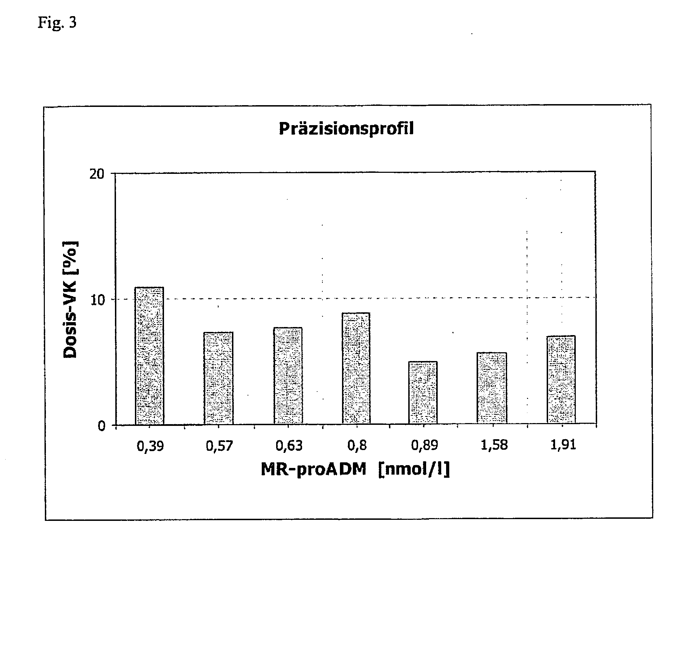

Das erfindungsgemäße Verfahren zeigt für MR-proADM eine hohe Präzision sowohl im Normbereich (Morgenthaler NG et al.: Clin Chem. 2005 Oct; 51(10):1823-9.) als auch im Bereich erhöhter Konzentrationen, die mit verschiedenen Krankheitsbildern (s.o.) assoziiert sind (Fig. 3).

Im Einzelnen wurde der Versuch wie folgt durchgeführt:

**D.1. Herstellung der Konjugate für die Festphase**

**D.1.1. Herstellung BSA+Oligo-Lys-Konjugat: siehe oben**

**D.1.2. Herstellung anti-"SPCD19" Schaf Antikörper biotinyliert+Oligo-Asp-Konjugat**

Zur Kopplung des Oligo-Asp Peptids "D14C" (Aminosäuresequenz DDDDDDDDDDDDDDC, (SEQ ID NO. 2), Firma JPT GmbH, Berlin, Deutschland, M = 1731,43) wurde ein polyclonaler anti-"SPCD19" (Aminosäuresequenz CRPQDMKGASRSPEDSSPD, (SEQ ID NO. 10) Firma JPT GmbH, Berlin, Deutschland, M = 2063) Schaf Antikörper mit SMCC im Verhältnis 1:25 aktiviert (siehe "Instructions SMCC and Sulfo-SMCC", Pierce, Rockford, IL, USA).

1,0 mg Antikörper wurden mit 16,67 $\mu$l 10 mM SMCC (frisch gelöst in DMSO) und 103 $\mu$l PBS, 10 mM EDTA, pH 8) versetzt und 30 min bei Raumtemperatur inkubiert. Das Produkt wurde über eine NAP5-Säule gereinigt (Laufmittel: PBS, 10 mM EDTA, pH 8) und der Proteingehalt photometrisch bestimmt.

Anschließend erfolgte sofort die Konjugation mit dem Oligo-Asp Peptid (gelöst in dest. Wasser) im molaren Verhältnis von 1:100. Nach Inkubation von 60 min bei Raumtemperatur wurde nicht konjugiertes Peptid über eine NAP10-Säule abgetrennt (Laufmittel: PBS, pH 7,4) und der Proteingehalt photometrisch bestimmt.

Das Produkt wurde mit EZ-Link Sulfo-NHS-LC-LC-Biotin (Firma Pierce, Rockford, IL, USA, Art. Nr. 21338) im molaren Verhältnis von 1:20 versetzt (siehe "Instructions EZ-Link SulfoNHS-LC-LC-Biotin", Pierce, Rockford, IL, USA): 900 $\mu$g Antikörper (in PBS pH 7,4) wurden mit 12 $\mu$l 10 mM EZ-Link Sulfo-NHS-LC-LC-Biotin (frisch gelöst in dest. Wasser) versetzt. Nach Inkubation von 30 min bei Raumtemperatur wurde die Reaktion mit 50 $\mu$l 1M Tris 10 min gestoppt. Das Produkt wurde über eine NAP25-Säule gereinigt (Laufmittel: PBS, pH 7,4) und der Proteingehalt photometrisch bestimmt.

Das anti-"SPCD19" Schaf Antikörper biotinyliert+Oligo-Asp-Konjugat wurde aliquotiert bei - 20°C gelagert.

**D.2. Kopplung des Konjugats auf Röhrchen**

**D.2.1. Kopplung: BSA+Oligo-Lys-Konjugat**

Bestrahlte Stemröhrchen (Firma Greiner, Frickenhausen, Deutschland) wurden mit BSA-Oligo-Lys-Konjugat wie folgt beschichtet:

Das Konjugat wurde in 10 mM Tris, 100 mM NaCl, pH 7,8 zu einer Konzentration von 6.67 $\mu$g/ml verdünnt. In jedes Röhrchen wurden 300 $\mu$l dieser Lösung pipettiert und 20 h bei 22°C inkubiert. Die Lösung wurde abgesaugt. Dann wurde jedes Röhrchen mit 300 $\mu$l 0,5% Bovines Serum Albumin, 2% Karion FP 2 h abgesättigt und nochmals abgesaugt. Anschließend erfolgte die Bindung des anti-Calcitonin Schaf Antikörper biotinyliert+Oligo-Asp-Konjugates:

**D.2.2. Bindung: anti-"SPCD19" Schaf Antikörper biotinyliert+Oligo-Asp-Konjugat**

Das Konjugat wurde in PBS, 0,5% proteasefreies BSA pH 7,4 zu einer Konzentration von 667 ng/ml verdünnt. In jedes Röhrchen wurden 300 $\mu$l dieser Lösung pipettiert und 1 Stunde bei 22°C inkubiert. Die Lösung wurde abgesaugt. Dann wurde jedes Röhrchen mit 300 $\mu$l 0,5% Bovines Serum Albumin, 2% Karion FP 2 h nochmals abgesättigt und danach abgesaugt.

Anschließend wurden die Röhrchen in einem Vakuumtrockner getrocknet und bei 4°C gelagert.

**D.3. Herstellung von Gold-markiertem anti-"PSV11" Antikörper**

Es wurde ein monoklonaler anti-"PSV11"-Antikörper verwendet (PSV11 stellt eine Teilsequenz von MR-proADM dar; Aminosäuresequenz CSSPDAARIRV, (SEQ ID NO. 11), Firma JPT GmbH, Berlin, Deutschland, M=1174). Der Antikörper wurde an kolloidales Gold (Firma BBI International, EM. GC 60nm) wie bei Hermanson beschrieben gekoppelt (http://www.amazon.de/Bioconjugate-Techniques-Greg-T-Hermanson/dp/0123423368).

**D.4. Herstellung der Teststreifen:**

Diese Komponente wurde von 8sens.biognostic GmbH, Berlin, Deutschland, erworben. Es wurden Nitrocellulose-Membranen von MDI (Advanced Microdevices, Ambala, Indien) mit 4 $\mu$g/cm Streptavidin (Pierce, Rockford, IL, USA) (Test-Linie) beschichtet. Zum Aufbringen der Fänger-Moleküle wurde ein Mikrodispenser der Firma Biodot, Irvine, CA, USA, eingesetzt. Anschließend erfolgte die Trocknung der Membran über Nacht bei 50°C. Als Saugpad kam AP22 der Firma Millipore, Billerica, MA, USA zum Einsatz.

**D.5. Immuntransfer und Immunchromatographie:**

Der im Folgenden beschriebene Assay wurde für jede Probe in 10-fach Bestimmung durchgeführt. In die mit BSA+Oligo-Lys und anti-"SPCD19" Schaf Antikörper biotinyliert+"D14C"-Konjugat beschichteten Röhrchen wurden 300 μl EDTA-Vollblut Proben, aufgestockt mit synthetischem 45-92 proAdrenomedullin (Aminosäuresequenz ELRMSSSYPTGLADVKAGPAQTLIRPQDMKGASRSPEDSSPDAARIRV, (SEQ ID NO. 9) Firma JPT GmbH, Berlin, Deutschland, M=5115) pipettiert und 15 min bei Raumtemperatur inkubiert (die proADM Konzentrationen der Proben wurden durch Messung mit dem Assay BRAHMS proADM LIA (BRAHMS AG, Hennigsdorf, Deutschland) aus Plasma bestimmt). Danach wurde 5x mit 1 ml Waschlösung (8 mM Tris, 60 mM NaCl, 0,2% Tween 20, pH 7,5) gewaschen.

Anschließend wurde mit 150 μl Ablösungspuffer (PBS pH 7,8, 0,5% BSA, 0,1% Tween 20, 0,1% Heparin, 0,08% NaN$_3$), der auch den Gold-markierten anti-"PSV11" monoklonalen Antikörper enthielt, unter Schütteln für 10 min inkubiert. Von dem abgelösten Immunkomplex wurden 100 μl in Kavitäten von low binding Mikrotiterplatten überführt. Anschliessend erfolgte unmittelbar die Immunchromatographie mit den Teststreifen, indem diese in die Kavitäten gesteckt wurden. Nach 30 min wurde die Färbung der Testbande mit einem Reflektrometer der Firma LRE Medical GmbH, München, Deutschland, bestimmt.

Aus den 10-fach Bestimmungen der einzelnen Proben wurde der jeweilige Mittelwert gebildet. Diese Werte dienten als Standardkonzentrationen, an denen die Konzentrationen aller einzelner Proben unter Verwendung der Software MultiCalc (Spline Fit) ermittelt wurden. Zu jeder 10- fach Bestimmung wurde der Variationskoeffizient bestimmt.

**Beschreibung der Figuren**

**Fig. 1:** zeigt, dass der Analyt (PCT) aus Vollblut immunextrahiert werden kann, und zwar bereits nach nur 5 min Inkubation mit ähnlicher Effizienz wie die Referenzmethode nach 2 h Inkubation.

**Fig. 2:** Dargestellt sind die Variationskoeffizienten für die Mehrfachbestimmung von Vollblutproben mit verschiedenen PCT-Konzentrationen, wie sie mit den drei folgend angegebenen Methoden ermittelt wurden (A: Immuntransfer mit TRACE Technologie, B: Immuntransfer mit Immunchromatographie, C: Referenz klassische Immunchromatographie). Beide Verfahren, die Immuntransfer enthalten, stellen erfindungsgemäße Verfahren dar; die klassische Immunchromatographie ist das Referenzverfahren.

**Fig. 3:** Dargestellt sind die Variationskoeffizienten für die Mehrfachbestimmung von Vollblutproben mit verschiedenen MR-proADM-Konzentrationen, wie sie mit der erfindungsgemäßen Methode Immuntransfer mit Immunchromatographie ermittelt wurden.

**Literaturliste**

**(1)**
M.J. Pugia, C.P. Price: Point-of-Care Testing, 2nd ed., edited by Price, John, Hicks, American Association for Clinical Chemistry, ISBN 1-59425-012-X, 2004; Seiten 13-30

**(2)**
Saadeddin S et al.: Reliability of the rapid bedside whole-blood quantitative cardiac troponin T assay in the diagnosis of myocardial injury in patients with acute coronary Syndrome, Med Sci Monit. 2004 Mar; 10(3):MT43-6. Epub 2004 Mar 1

**(3)**
o. V. - Evaluation of a bedside whole-blood rapid troponin T assay in the emergency department. Rapid Evaluation by Assay of Cardiac Troponin T (REACTT) Investigators Study Group, Acad Emerg Med. 1997 Nov; 4(11):1018-24

**(4)**
Zugck C et al.: Multicentre evaluation of a new point-of-care test for the determination of NT-proBNP in whole blood, Clin Chem Lab Med. 2006; 44(10): 1269-77

**(5)**

Alan H. B.: A Platform for Quantitative Point-of-Care Cardiac Marker Determinations from Roche Diagnostics

**(6)**
Yeo KT et al.: Multicenter evaluation of the Roche NT-proBNP assay and comparison to the Biosite Triage BNP assay, Clin Chim Acta. 2003 Dec; 338(1 -2): 107- 15

**(7)**
Meisner M et al.: Clinical experiences with a new semi-quantitative solid phase immunoassay for rapid measurement of procalcitonin, Clin Chem Lab Med. 2000 Oct; 38(10):989-95

**(8)**
Terpe K.: Overview of tag protein fusions: from molecular and biochemical fundamentals to commercial Systems, Appl Microbiol Biotechnol. 2003 Jan; 60(5):523-33. Epub 2002 Nov 7

**(9)**
R.C. Stevens: Fusion tags used in recombinant protein expression and purification - Design of high-throughput methods of protein production for structural biology, Structure, 8, R177-R185 (2000).

**(10)**
Stevens RC: Design of high-throughput methods of protein production for structural biology Structure. 2000 Sep 15; 8(9):R177-85

**(11)**
Hopp TP, Gallis B, Prickett KS: Metal-binding properties of a calcium-dependent monoclonal antibody., Mol Immunol. 1996 May-Jun; 33(7-8):601-8

**(12)**
Steams DJ et al.: The interaction of a Ca2+-dependent monoclonal antibody with the protein C activation peptide region. Evidence for obligatory Ca2+ binding to both antigen and antibody, J Biol Chem. 1988 Jan 15; 263(2):826-32

**(13)**
QIAexpress Detection and Assay Handbook 10/2002, QIAGEN, HILDEN, DEUTSCHLAND

**(14)**
Sassenfeld,H.M. and Brewer,S J. (1984) Bio/Technology, 2, 76-80.

**(15)**
Dalboge,H., Dahl,H.-H.M., PedersenJ., Hansen, J.W. and Christensen,T. (1987), Bio/Technology, 5, 161-164

**(16)**
Zhao,J.Y., Ford,C.F., Glatz,C.E., Rougvie,M.A. and Gendel.S.M. (1990) J. Biotechnol., 14, 273-283

**(17)**
Avidin-Biotin Chemistry: A Handbook

**(18)**
Invitrogen (Bibliothek)

**(19)**
Pierce: Immobilized Monomeric Avidin Kit, www.piercenet.com

**(20)**
Pierce: Two thiol cleavable, heterobifunctional, amine- and sulfhydryl-reactive crosslinking agents, www.piercenet.com

**(21)**
Meisner M.: Pathobiochemistry and clinical use of procalcitonin, Clin Chim Acta. 2002 Sep;323(1-2): 17-29

EP 2 167 965 B1

**(22)**

Meisner M et al.: Clinical experiences with a new semi-quantitative solid phase immunoassay for rapid measurement of procalcitonin, Clin Chem Lab Med. 2000 Oct;38(10):989-95

**(23)**

Morgenthaler NG et al.: Sensitive immunoluminometric assay for the detection of procalcitonin, Clin Chem. 2002 May;48(5):788-90

## Patentansprüche

1. Verfahren zur Detektion von Analyten aus biologischen Proben umfassend die folgenden Verfahrensschritte:

   **a)** Bereitstellung eines an eine Festphase immobilisierten Reversiblen Bindungspartners 1, an den ein Analyt-Binder über einen an den Analyt-Binder gebundenen Reversiblen Bindungspartner 2 reversibel gebunden ist, wobei der Analyt-Binder immobilisiert wird durch Bindung zwischen den Reversiblen Bindungspartnern 1 und 2,
   **b)** Hinzugabe der biologischen Probe und Bindung des Analyten an den reversibel immobilisierten Analyt-Binder im Fall, dass die biologische Probe den Analyten enthält,
   **c)** Abtrennung der biologischen Probe,
   **d)** Zugabe eines Ablösepuffers, der die Bindung zwischen den Reversiblen Bindungspartnern 1 und 2 löst, wobei die Bindung des Analyten an den Analyt-Binder optional bestehen bleibt, und wobei der Ablösepuffer eine weitere für die Detektion erforderliche markierte Komponente enthält, die ein zweiter Analyt-Binder für die Durchführung eines Sandwichimmunoassays ist, und
   **e)** Detektion des Analyten im Ablösepuffer im Fall, dass die biologische Probe den Analyten enthält beziehungsweise Feststellen der Abwesenheit des Analyten im Fall, dass die biologische Probe den Analyten nicht enthält.

2. Verfahren gemäß Anspruch 1, wobei die biologische Flüssigkeit eine unprozessierte Vollblutprobe ist.

3. Verfahren gemäß einem der Ansprüche 1 oder 2, wobei der immobilisierte Reversible Bindungspartner 1 direkt oder mittels eines Trägerproteins immobilisiert ist.

4. Verfahren gemäß einem der Ansprüche 1 bis 3, wobei der Analyt-Binder kovalent an den Reversiblen Bindungspartner 2 gebundenen ist.

5. Verfahren gemäß einem der Ansprüche 1 bis 4, wobei der Analyt-Binder ein Anti-Procalcitonin Antikörper ist.

6. Verfahren gemäß einem der Ansprüche 1 bis 5, wobei das Bindungspaar Reversibler Bindungspartner 1 und 2 ausgewählt ist aus einem der folgenden Bindungspaare:

   **a)** positiv und negativ geladene Peptidoligomere,
   **b)** $Ca^{2+}$-bindendes Peptid/Protein und Antikörper, der das Peptid/Protein mit grö-βerer Affinität bindet, wenn das Peptid/Protein $Ca^{2+}$ gebunden hat,
   **c)** Oligohistidin (z.B. 6His) und Ni-NTA,
   **d)** Biotin und Avidin oder Streptavidin oder Neutravidin.

7. Verfahren gemäß einem dem Anspruch 6, wobei das $Ca^{2+}$-bindende Peptid/Protein ein FLAG-Peptid und der Antikörper ein M1 Antikörper ist oder das $Ca^{2+}$-bindende Peptid/Protein ein Protein C Peptid und der Antikörper ein HPC4 Antikörper ist.

8. Verfahren gemäß einem der Ansprüche 1 bis 7, wobei der Analyt-Binder markiert ist und auch nach Zugabe des Ablösepuffers markiert bleibt mit einem Label für die Detektion des Analyten.

9. Verfahren gemäß einem der Ansprüche 1 bis 8, wobei die Detektion des markierten Analyten durch einen Immunoassay unter Verwendung der TRACE Technologie erfolgt.

10. Verfahren gemäß einem der Ansprüche 1 bis 9, wobei die Detektion des markierten Analyten mittels Immunochromatographie erfolgt.

**11.** Verfahren gemäß einem der Ansprüche 1 bis 10, wobei die biologische Probe unverdünnt und in einem solchen Volumen eingesetzt wird, dass der beschichtete Anteil der Festphase vollständig in Kontakt mit der biologischen Probe gebracht wird.

**12.** Verfahren gemäß einem der Ansprüche 1 bis 11, wobei die Dauer von Zugabe der Probe bis zur Detektion 30 min nicht überschreitet und das Verfahren als Schnelltestverfahren anzusehen ist.

**13.** Kit zur Durchführung eines Verfahrens gemäß einem der Ansprüche 1 bis 12 umfassend:

**a)** eine Festphase mit einem immobilisierten Reversiblen Bindungspartner 1,
**b)** Komplex umfassend Analyt-Binder gebunden an den Reversiblen Bindungspartner 2, wobei dieser Komplex optional bereits durch Bindung der Reversiblen Bindungspartner 1 und 2 auf der Festphase immobilisiert vorliegen kann,
**c)** Ablösepuffer, der die Bindung zwischen den Reversiblen Bindungspartnern 1 und 2 löst, wobei jedoch die Bindung des Analyten an den Analyt-Binder optional bestehen bleibt, und wobei der Ablösepuffer eine weitere für die Detektion erforderliche markierte Komponente enthält, die ein zweiter Analyt-Binder für die Durchführung eines Sandwichimmunoassays ist.

**14.** Kit gemäß Anspruch 13, wobei der Analyt-Binder ein Anti-PCT Antikörper ist.

**15.** Kit gemäß Anspruch 13 oder 14, wobei das Bindungspaar Reversibler Bindungspartner 1 und 2 ausgewählt ist aus einem der folgenden Bindungspaare:

**a)** positiv und negativ geladene Peptidoligomere,
**b)** $Ca^{2+}$-bindendes Peptid/Protein und Antikörper, der das Peptid/Protein mit größerer Affinität bindet, wenn das Peptid/Protein $Ca^{2+}$ gebunden hat,
**c)** Oligohistidin (z.B. 6His) und Ni-NTA,
**d)** Biotin und Avidin oder Streptavidin oder Neutravidin.

**Claims**

**1.** A method to detect analytes from biological samples comprising the following method steps:

**a)** Providing a Reversible Binding Partner 1 immobilized in a solid phase to which an analyte binder is reversibly bound via a Reversible Binding Partner 2, which is itself bound to the analyte binder, where the analyte binder is immobilized by way of the bond between the Reversible Binding Partners 1 and 2.
**b)** Adding the biological sample and bonding the analyte to the reversibly immobilized analyte binder in the event that the biological sample contains the analytes,
**c)** Separating the biological sample,
**d)** Adding a dissolution buffer that breaks the bonds between the Reversible Binding Partners 1 and 2, whereby it is optional whether the analyte remains bonded to the analyte binder, and whereby the dissolution buffer contains another marked component required for detection, namely a second analyte binder used to perform a sandwich immunoassay, and
**e)** Detecting the analyte in the dissolution buffer in the event that the biological sample contains the analyte, or determining whether the analyte is absent in the event that the biological sample does not contain the analyte, respectively.

**2.** The method according to Claim 1, wherein the biological fluid is an unprocessed sample of whole blood.

**3.** The method according to any of Claims 1 or 2, wherein the Immobilized Reversible Binding Partner 1 is immobilized directly or by way of a carrier protein.

**4.** The method according to any of Claims 1 to 3, wherein the analyte binder is bonded covalently to the Reversible Binding Partner 2.

**5.** The method according to any of Claims 1 to 4, wherein the analyte binder is an anti-procalcitonin antibody.

6. The method according to any of Claims 1 to 5, wherein the bonded pair of Reversible Binding Partners 1 and 2 is selected from any of the following bonding pairs:

a) positively and negatively charged peptide oligomeres,
b) $Ca^{2+}$-binding peptide/protein and antibody, which latter bonds the peptide/protein with high affinity when the peptide/protein has bonded with $Ca^{2+}$,
c) Oligohistidine (e.g. 6His) and Ni-NTA,
d) Biotin and avidin or streptavidin or neutravidin.

7. The method according to any of Claims 1 to 6, wherein the $Ca^{2+}$-binding peptide/protein is a FLAG peptide and the antibody is an M1 antibody, or the $Ca^{2+}$-binding peptide/protein is a C-peptide protein and the antibody is an HPC4 antibody.

8. The method according to any of Claims 1 to 7, wherein the analyte binder is marked and remains marked with a label for detecting the analyte even after addition of the dissolution buffer.

9. The method according to any of Claims 1 to 8, wherein the marked analyte is detected using an immunoassay using TRACE technology.

10. The method according to any of Claims 1 to 9, wherein the marked analyte is detected using immunochromatography.

11. The method according to any of Claims 1 to 10, wherein the biological sample is undiluted and used in such a volume that the coated part of the solid state is brought completely into contact with the biological sample.

12. The method according to any of Claims 1 to 11, wherein the amount of time from addition of the sample to detection does not exceed 30 minutes and the method is regarded as a quick-test method.

13. A kit to carry out a method according to any of Claims 1 to 12, comprising:

a) a solid phase with an immobilized Reversible Binding Partner 1,
b) a complex comprising analyte binder bonded to the Reversible Binding Partner 2, whereby this complex can optionally already be immobilized on the solid phase by bonding of the Reversible Binding Partners 1 and 2.
c) a dissolution buffer that breaks the bond between the Reversible Binding Partners 1 and 2, whereby the bond between the analyte and the analyte binder may, however, remain intact, and whereby the dissolution buffer contains another marked component required for detection, namely a second analyte binder for performing a sandwich immunoassay.

14. The kit according to Claim 13, whereby the analyte binder is an anti-PCT antibody.

15. The kit according to Claim 13 or 14, wherein the bonding pair of Reversible Binding Partners 1 and 2 is selected from one of the following bonding pairs:

a) positively and negatively charged peptide oligomeres,
b) $Ca^{2+}$-binding peptide/protein and antibody, which latter bonds the peptide/protein with high affinity when the peptide/protein has bound with $Ca^{2+}$,
c) Oligohistidine (e.g. 6His) and Ni-NTA,
d) Biotin and avidin or streptavidin or neutravidin.

**Revendications**

1. Procédé de détection d'analytes d'échantillons biologiques, comprenant les étapes de procédé suivants :

a) mise à disposition d'un partenaire de liaison réversible 1 immobilisé sur une phase solide, partenaire de liaison auquel est lié de manière réversible un liant d'analyte via un partenaire de liaison réversible lié au liant d'analyte, étant donné que le liant d'analyte est immobilisé par liaison entre les partenaires de liaison réversibles 1 et 2,
b) ajout de l'échantillon biologique et liaison de l'analyte au liant d'analyte immobilisé de manière réversible dans le cas où l'échantillon biologique contient l'analyte,

c) séparation de l'échantillon biologique,

d) ajout d'une solution tampon de séparation qui sépare la liaison entre le partenaires de liaison réversibles 1 et 2, étant donné que la liaison de l'analyte au liant d'analyte est en option maintenue, et étant donné que la solution tampon de séparation contient une autre composante marquée requise pour la détection, composante qui est un deuxième liant d'analyte pour l'exécution d'un test immunologique de type Sandwich, et

e) détection de l'analyte dans la solution tampon de séparation dans le cas où l'échantillon biologique contient l'analyte, ou constatation de l'absence de l'analyte dans le cas où l'échantillon biologique ne contient pas l'analyte.

2. Procédé selon la revendication 1, étant donné que le liquide biologique est un échantillon de sang entier non traité.

3. Procédé selon l'une quelconque des revendications 1 ou 2, étant donné que le partenaire de liaison réversible immobilisé 1 est immobilisé directement ou au moyen d'une protéine porteuse.

4. Procédé selon l'une quelconque des revendications 1 à 3, étant donné que le liant d'analyte est lié de manière covalente au partenaire de liaison réversible 2.

5. Procédé selon l'une quelconque des revendications 1 à 4, étant donné que le liant d'analyte est un anticorps anti-procalcitonine.

6. Procédé selon l'une quelconque des revendications 1 à 5, étant donné que la paire de liaison constituée par les partenaires de liaison réversibles 1 et 2 est sélectionnée parmi l'une des paires de liaison suivantes :

a) oligomères peptidiques à charge positive et négative,

b) peptide/protéine liant le $Ca^{2+}$ et anticorps qui lie le peptide / la protéine avec une plus grande affinité lorsque le peptide / la protéine a lié le $Ca^{2+}$,

c) oligohistidine (p. ex. 6His) et Ni-NTA,

d) biotine et avidine ou streptavidine ou neutravidine.

7. Procédé selon la revendication 1 à 6, étant donné que peptide / la protéine liant le $Ca^{2+}$ est un peptide FLAG et l'anticorps est un anticorps MI ou le peptide / la protéine liant le $Ca^{2+}$ est un peptide de protéine C et l'anticorps est un anticorps HPC4.

8. Procédé selon l'une quelconque des revendications 1 à 7, étant donné que le liant d'analyte est marqué et reste marqué, même après l'ajout de la solution tampon de séparation, par un label pour la détection de l'analyte.

9. Procédé selon l'une quelconque des revendications 1 à 8, étant donné que la détection de l'analyte marqué se fait par un test immunologique utilisant la technologie TRACE.

10. Procédé selon l'une quelconque des revendications 1 à 9, étant donné que la détection de l'analyte marqué se fait par immunochromatographie.

11. Procédé selon l'une quelconque des revendications 1 à 10, étant donné que l'échantillon biologique est utilisé non dilué et en un volume tel que la partie revêtue de la phase solide soit complètement mise en contact avec l'échantillon biologique.

12. Procédé selon l'une quelconque des revendications 1 à 11, étant donné que la durée qui s'écoule de l'ajout de l'échantillon jusqu'à la détection ne dépasse pas 30 mn et le procédé est considéré comme procédé de test rapide.

13. Kit pour la réalisation d'un procédé selon l'une quelconque des revendications 1 à 12, comprenant :

a) une phase solide avec un partenaire de liaison réversible immobilisé 1,

b) un complexe comprenant un liant d'analyte lié au partenaire de liaison réversible 2, dans lequel étant donné que ce complexe peut en option déjà être présent immobilisé par liaison des partenaires de liaison réversibles 1 et 2 sur la phase solide,

c) une solution tampon de séparation qui sépare la liaison entre le partenaires de liaison réversibles 1 et 2, étant donné toutefois que la liaison de l'analyte au liant d'analyte est en option maintenue, et étant donné que la solution tampon de séparation contient une autre composante marquée requise pour la détection, composante qui est un deuxième liant d'analyte pour l'exécution d'un test immunologique de type Sandwich.

14. Kit selon la revendication 13, étant donné que le liant d'analyte est anticorps Anti-PCT.

15. Kit selon la revendication 13 ou 14, étant donné que la paire de liaison constituée par les partenaires de liaison réversibles 1 et 2 est sélectionnée parmi l'une des paires de liaison suivantes :

a) oligomères peptidiques à charge positive et négative,
b) peptide/protéine liant le $Ca^{2+}$ et anticorps qui lie le peptide / la protéine avec une plus grande affinité lorsque le peptide / la protéine a lié le $Ca^{2+}$,
c) oligohistidine (p. ex. 6His) et Ni-NTA,
d) biotine et avidine ou streptavidine ou neutravidine.

Fig. 1

Fig. 2

# Präzisionsprofile (Dosis-VK)

● Immunchromatograpie

O Immuntransfer + Immunchromatographie

✕ Immuntransfer + TRACE Assay

Fig. 3

**Präzisionsprofil**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4851341 A

**In der Beschreibung aufgeführte Nicht-Patentliteratur**

- **FRENS, G.** *Nature Physical Science,* 1973, vol. 241, 20-22
- **STRUCK J et al.** *Peptides,* August 2004, vol. 25 (8), 1369-72
- **BELTOWSKI J et al.** *Pol J Pharmacol.,* Januar 2004, vol. 56 (1), 5-27
- **STOLZ D et al.** *Chest,* 19. Mai 2008
- **CHRIST-CRAIN M et al.** *Crit Care,* 2006, vol. 10 (3), R96
- **CHRIST-CRAIN M et al.** *Crit Care,* 2005, vol. 9 (6), R816-24
- **KHAN SQ et al.** *J Am Coli Cardiol.,* 10. April 2007, vol. 49 (14), 1525-32
- **GEGENHUBER A et al.** *J Card Fail.,* Februar 2007, vol. 13 (1), 42-9
- **MORGENTHALER NG et al.** *Clin Chem.,* Oktober 2005, vol. 51 (10), 1823-9
- **M.J. PUGIA ; C.P. PRICE.** Point-of-Care Testing. American Association for Clinical Chemistry, 2004, 13-30
- **SAADEDDIN S et al.** Reliability of the rapid bedside whole-blood quantitative cardiac troponin T assay in the diagnosis of myocardial injury in patients with acute coronary Syndrome. *Med Sci Monit.,* 01. Marz 2004, vol. 10 (3), MT43-6
- V. - Evaluation of a bedside whole-blood rapid troponin T assay in the emergency department. Rapid Evaluation by Assay of Cardiac Troponin T (REACTT) Investigators Study Group. *Acad Emerg Med.,* November 1997, vol. 4 (11), 1018-24
- **ZUGCK C et al.** Multicentre evaluation of a new point-of-care test for the determination of NT-proBNP in whole blood. *Clin Chem Lab Med.,* 2006, vol. 44 (10), 1269-77
- **ALAN H. B.** *A Platform for Quantitative Point-of-Care Cardiac Marker Determinations from Roche Diagnostics*
- **YEO KT et al.** Multicenter evaluation of the Roche NT-proBNP assay and comparison to the Biosite Triage BNP assay. *Clin Chim Acta,* Dezember 2003, vol. 338 (1 -2), 107-15
- **MEISNER M et al.** Clinical experiences with a new semi-quantitative solid phase immunoassay for rapid measurement of procalcitonin. *Clin Chem Lab Med.,* Oktober 2000, vol. 38 (10), 989-95
- **TERPE K.** Overview of tag protein fusions: from molecular and biochemical fundamentals to commercial Systems. *Appl Microbiol Biotechnol.,* 07. November 2002, vol. 60 (5), 523-33
- **R.C. STEVENS.** Fusion tags used in recombinant protein expression and purification - Design of high-throughput methods of protein production for structural biology. *Structure,* 2000, vol. 8, R177-R185
- **STEVENS RC.** Design of high-throughput methods of protein production for structural biology. *Structure,* 15. September 2000, vol. 8 (9), R177-85
- **HOPP TP ; GALLIS B ; PRICKETT KS.** Metal-binding properties of a calcium-dependent monoclonal antibody. *Mol Immunol.,* Mai 1996, vol. 33 (7-8), 601-8
- **STEAMS DJ et al.** The interaction of a Ca2+-dependent monoclonal antibody with the protein C activation peptide region. Evidence for obligatory Ca2+ binding to both antigen and antibody. *J Biol Chem.,* 15. Januar 1988, vol. 263 (2), 826-32
- QIAexpress Detection and Assay Handbook. Oktober 2002
- **SASSENFELD,H.M. ; BREWER,S J.** *Bio/Technology,* 1984, vol. 2, 76-80
- **DALBOGE,H. ; DAHL,H.-H.M ; PEDERSENJ. ; HANSEN, J.W. ; CHRISTENSEN,T.** *Bio/Technology,* 1987, vol. 5, 161-164
- **ZHAO,J.Y. ; FORD,C.F ; GLATZ,C.E. ; ROUGVIE,M.A. ; GENDEL.S.M.** *J. Biotechnol.,* 1990, vol. 14, 273-283
- Avidin-Biotin Chemistry: A Handbook
- **MEISNER M.** Pathobiochemistry and clinical use of procalcitonin. *Clin Chim Acta,* September 2002, vol. 323 (1-2), 17-29
- **MORGENTHALER NG et al.** Sensitive immunoluminometric assay for the detection of procalcitonin. *Clin Chem.,* Mai 2002, vol. 48 (5), 788-90